# EUROPÄISCHE PATENTSCHRIFT

(11) **EP 0 602 514 B1**
(45) Veröffentlichungstag und Bekanntmachung des Hinweises auf die Patenterteilung: **24.09.1997**
(21) Anmeldenummer: 93119728.9
(22) Anmeldetag: 08.12.1993
(51) Int. Cl.: C07C 327/44, A01N 37/50

(54) **Thioamide und deren Verwendung als Pflanzenschutzmittel**
Thioamides and their use as plant-protection agents
Thioamides et leur utilisation comme agents phytosanitaires

(30) Priorität: 14.12.1992 DE 4242081
(43) Veröffentlichungstag der Anmeldung: 22.06.1994
(73) Patentinhaber: BASF Aktiengesellschaft, 67063 Ludwigshafen (DE)
(72) Erfinder: Wingert, Horst, Dr., D-68159 Mannheim (DE); Sauter, Hubert, Dr., D-68167 Mannheim (DE); Bayer, Herbert, Dr., D-68159 Mannheim (DE); Oberdorf, Klaus, Dr., D-69117 Heidelberg (DE); Lorenz, Gisela, Dr., D-67434 Neustadt (DE); Ammermann, Eberhard, Dr., D-64646 Heppenheim (DE)

(56) Entgegenhaltungen:
- EP-A- 0 280 185
- EP-A- 0 398 692
- EP-A- 0 432 503
- EP-A- 0 477 631
- EP-A- 0 528 681
- TETRAHEDRON LETTERS, Nr. 16 , 1977 Oxford, GB, Seiten 1351 - 1354 E. SCHAUMANN, ET AL.: 'Konkurenz zwischen 1,2- und 1,3-Ringöffnung in der Umsetzung von 3-Dimethylamino-2-phenyl-2H-aziridinen mit Kohlenstoffdisulfid'
- COMPTES RENDUS HEBDOMADAIRES DES SEANCES DE L'ACADEMIE DES SCIENCES, SERIE C, Bd. 268, Nr. 3 , 20. Januar 1969 Montreuil, FR, Seiten 294 - 296 J. CHAUVIN, ET AL.: 'Propriétés de N,N-diméthylthioamides'

## Beschreibung

Die vorliegende Erfindung betrifft neue Thioamide, Verfahren zu deren Herstellung und Verfahren zur Bekämpfung von Schädlingen, insbesondere von Pilzen, Insekten, Nematoden und Spinnmilben mit diesen Verbindungen.

Es ist bekannt, daß bestimmte Thiocarbonsäureester (EP 432 503), bestimmte O-Benzyl-Oximether (EP 463 488), oder bestimmte Phenylessigsäureamide (vgl. EP 477 631, 398 692) fungizide oder insektizide Wirkung besitzen. Ihre Wirkung ist jedoch unbefriedigend.

Daneben sind für die Vertragsstaaten BE, CH, DE, DK, FR, GB, IE, IT, NL und SE in der nachveröffentlichten EP-A 528 681 bestimmte Phenylmethoximinoverbindungen mit fungizider und insektizider Wirkung beschrieben.

Es wurde nun überraschend gefunden, daß die neuen Thioamide der allgemeinen Formel I 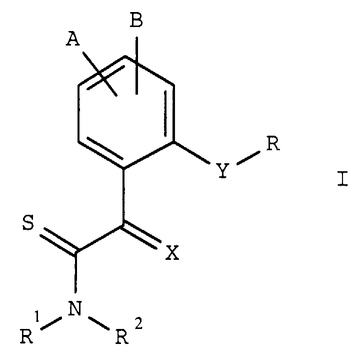 in der
- A, B, R': unabhängig voneinander Wasserstoff, Cyano, C₁-C₄-Alkyl, C₁-C₄-Alkoxy oder Halogen bedeuten,
- X: =CHCH₃ oder =N-OCH₃ bedeutet,
- R¹, R²: Wasserstoff oder C₁-C₄-Alkyl bedeuten,
- Y: für die Gruppierung
-O-, -S-, 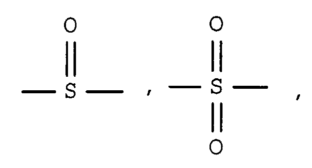 -O-CH₂, -CH₂-O-, -S-CH₂-, -CH₂-S-, -CH₂-CH₂, -CH=CH-, -C≡C-, -CH₂-O-N=C(R')- steht und
- R: Wasserstoff, ggf. subst. C₁-C₁₀-Alkyl, ggf. subst. C₁-C₆-Cycloalkyl, ggf. subst. Aryl oder ggf. subst. Hetaryl bedeutet, wobei der Ausdruck "ggf. subst." neben Wasserstoff auch Halogen, Cyano, CO₂(C₁-C₄-Alkyl) C(O)(C₁-C₄-Alkyl), Nitro, C₁-C₄-Alkyl, C₁-C₄-Alkoxy, C₁-C₄-Halogenalkyl, C₁-C₄-Alkoximino-C₁-C₄-alkyl, Aryl, Aryloxy, Benzyloxy, Hetaryl, Hetaryloxy, C₃-C₆-Cycloalkyl bedeutet und der Ausdruck "Hetaryl" für einen ggf. subst. aromatischen, ein-, zwei- oder dreikernigen Fünfring- oder Sechsring-Heterocyclus steht,
eine ausgezeichnete fungizide, insektizide, nematizide und akarizide Wirkung haben.

Die in der allgemeinen Formel I aufgeführten Reste können beispielsweise die folgende Bedeutung haben:
- A, B, R': können Wasserstoff, Cyano, C₁-C₄-Alkyl (z.B. Methyl), C₁-C₄-Alkoxy (z.B. Methoxy), Halogen (Fluor, Chlor, Brom, Jod) bedeuten,
- X: kann =CHCH₃ und =N-OCH₃ bedeuten,
- R¹, R²: können Wasserstoff und C₁-C₄-Alkyl (z.B. Methyl, Ethyl) bedeuten
- Y: steht für die Gruppierung
-O-, -S-, 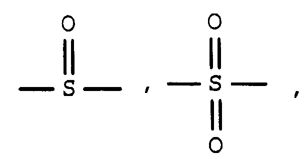 -O-CH₂, -CH₂-O-, -S-CH₂-, -CH₂-S-, -CH₂-CH₂, -CH=CH-, -C≡C-, -CH₂-O-N=C(R')- und
- R: kann Wasserstoff, ggf. subst. C₁-C₁₀-Alkyl (z.B. Methyl, Ethyl, n-, i-Propyl, n-, i-, s-,tert.-Butyl, Trifluormethyl, Cyanomethyl, Benzyl, Methoxymethyl), ggf. subst. C₁-C₆-Cycloalkyl (z.B. Cyclopropyl, Cyclobutyl, Cyclopentyl, Cyclohexyl, 1-Methyl-cyclopropyl), ggf. subst. Aryl (z.B. Phenyl, 2-Methylphenyl, 3-Methoxyphenyl, 4-tert.-Butoxy-phenyl, Naphthyl, 1-Methyl-2-naphthyl), ggf. subst. Hetaryl (z.B. Pyridinyl, 6-Cl-pyridin-2-yl, Thiazolyl, 2-Benzthiazolyl, Furyl, 2-Furyl, Pyrimidinyl, 4-Pyrimidinyl) bedeuten, wobei der Ausdruck "ggf. subst" neben Wasserstoff auch Halogen (z.B. Fluor, Chlor, Brom, Jod), Cyano, CO₂(C₁-C₄-Alkyl) (z.B. CO₂Me, CO₂Et), C(O)(C₁-C₄-Alkyl) (z.B. C(O)CH₃), Nitro, C₁-C₄-Alkyl (z.B. Methyl, Ethyl, n-, iso-Propyl, n-, i-,s-, tert.-Butyl), C₁-C₄-Alkoxy (z.B. Methoxy, Ethoxy, n-, iso-Propoxy, n-, i-, s-, tert.-Butoxy), C₁-C₄-Halogenalkyl (z.B. Chlormethyl, Brommethyl, Trifluormethyl, 1,1,2,2-Tetrafluorethyl), C₁-C₄-Alkoximino-C₁-C₄-alkyl (z.B. Methoximinomethyl, Ethoximinomethyl, n-, iso-Propoximinomethyl, n-, i-, s-, tert.-Butoximino-methyl, Methoximinoethyl), Aryl (z.B. Phenyl, 1-, 2-Naphthyl), Aryloxy (Phenoxy, 2-Naphthyloxy), Benzyloxy, Hetaryl (z.B. Pyridyl, Pyrimidinyl, Pyridazinyl, Chinolinyl, Furyl, Thienyl, Oxazolyl, Isoxazolyl, Benzthiazolyl, Benzthienyl, Benzoxazolyl, Pyrryl), Hetaryloxy (z.B. 2-Pyridinyloxy, 2-Chinolinyloxy, 4-Pyrimidinyloxy), C₃-C₆-Cycloalkyl (z.B. Cyclopropyl, Cyclobutyl, Cyclopentyl, Cyclohexyl) bedeutet und der Ausdruck "Hetaryl" für einen ggf. subst. aromatischen ein-, zwei oder dreikernigen Fünfring- oder Sechsring-Heterocyclus steht (z.B. Pyridin, Chinolin, Pyrimidin, Benzthiazol, Benzoxazol) steht.

Bevorzugt sind Verbindungen der allgemeinen Formel I 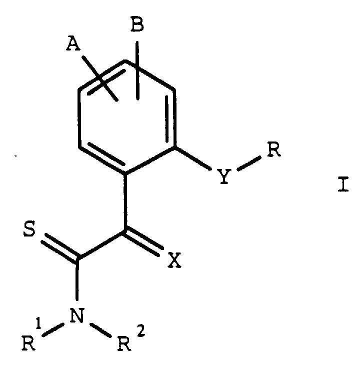 in der
- A, B,: Wasserstoff bedeuten,
- X: =N-OMe bedeutet,
- R¹: Wasserstoff bedeutet,
- R²: Methyl bedeutet,
und Y, R' und R die in Anspruch 1 aufgeführte Bedeutung haben.

Die neuen Verbindungen der allgemeinen Formel I können bei der Herstellung aufgrund der C=C- bzw. C=N-Doppelbindungen als E/Z-Isomerengemische anfallen. Diese können in der üblichen Weise, z.B. durch Kristallisation oder Chromatographie in die einzelnen Komponenten aufgetrennt werden. Sowohl die einzelnen isomeren Verbindungen als auch ihre Gemische werden von der Erfindung umfaßt und sind als Schädlingsbekämpfungsmittel brauchbar.

Die Darstellung der Thioamide der allgemeinen Formel I erfolgt beispielsweise indem man die Amide der allgemeinen Formel II in an sich bekannter Weise (vgl. z.B. Houben-Weyl, Bd IX, S. 764ff) mit einem Schwefelungsreagenz, wie z.B. P₄S₁₀ oder dem Lawesson-Reagenz 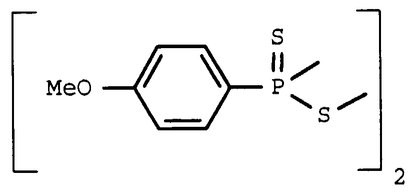 umsetzt.

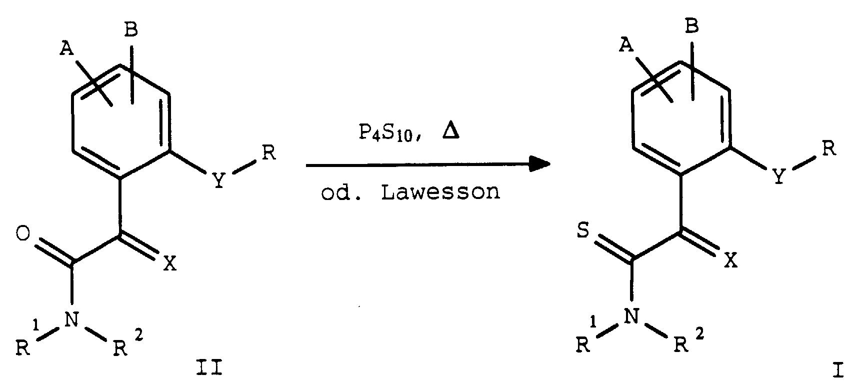

Die Amide II sind bekannt oder können in Analogie zu bekannten Methoden dargestellt werden (vgl. EP 477631, 398692, 463488).

Die folgenden Beispiele erläutern die Herstellung der Verbindungen.

### Beispiel 1:

Darstellung von 2-Methoximino-2-[2'-(o-methylphenoxymethyl)-phenyl]essigsaure-N-Methylthioamid (Verbindung 2, Tabelle 1).

5 g (16 mmol) 2-Methoximino-2-[2'-(o-methylphenoxyethyl)-phenyl]essigsäsure-N-Methylamid (bekannt aus EP 477631) und 8,1 g (20 mmol) Lawesson-Reagenz werden in 50 ml Xylol 90 min unter Rückfluß erhitzt. Anschließend wird das Lösungsmittel im Vakuum entfernt. Zweifache Chromatographie des Rückstandes an Kieselgel mit Hexan/Methyl-tert.-butylether-Gemischen liefert 1,7 g (32 %) der Verbindung als gelbes Öl.

¹H-NMR (CDCl₃/TMS): δ = 2,25 (CH₃); 3,21 (d, NHCH₃), 3,95 (OCH₃); 4,97 (CH₂); 6,79 - 7,55 (Aryl, 8H); 8,57 (NH).

Die in den folgenden Tabellen aufgeführten Verbindungen können analog dargestellt werden:

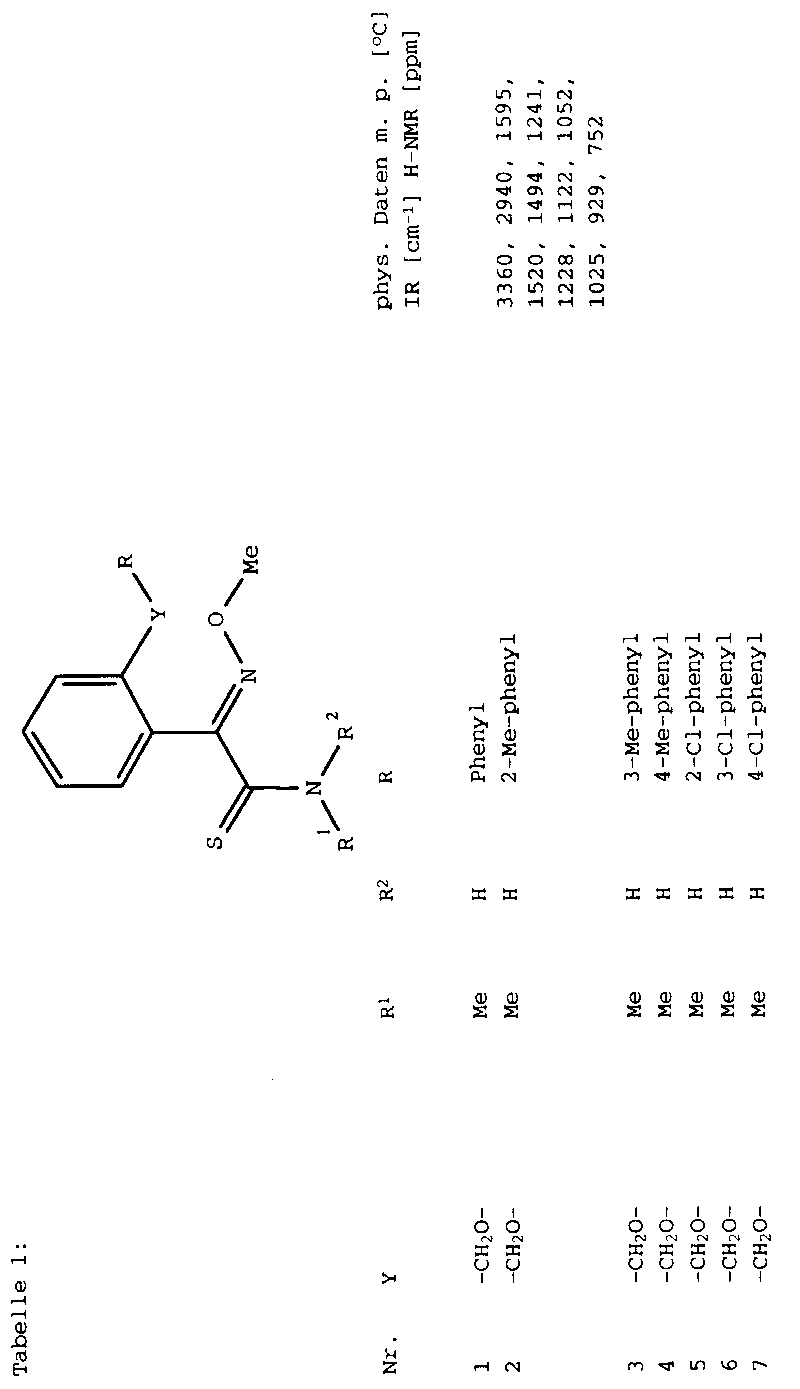

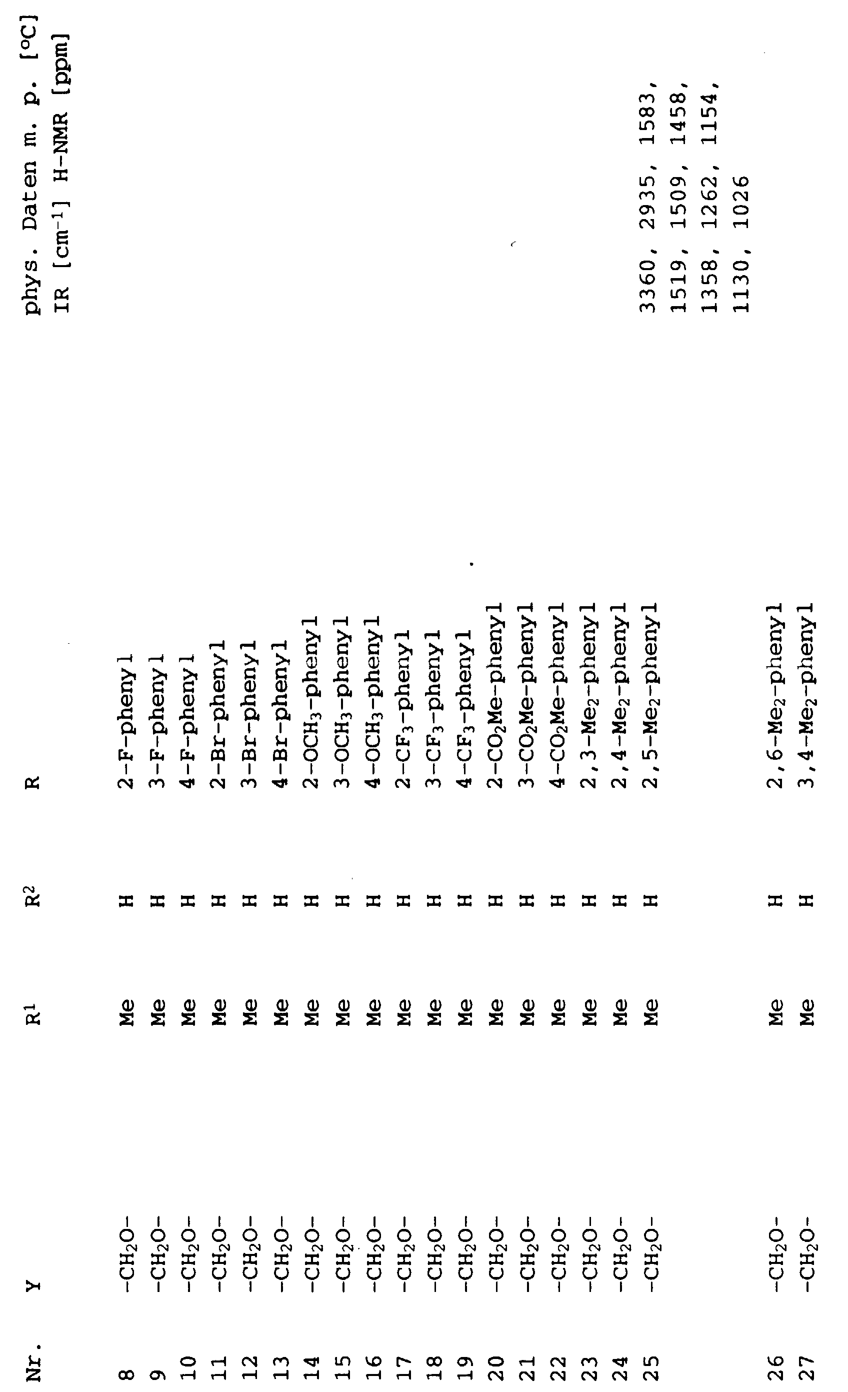

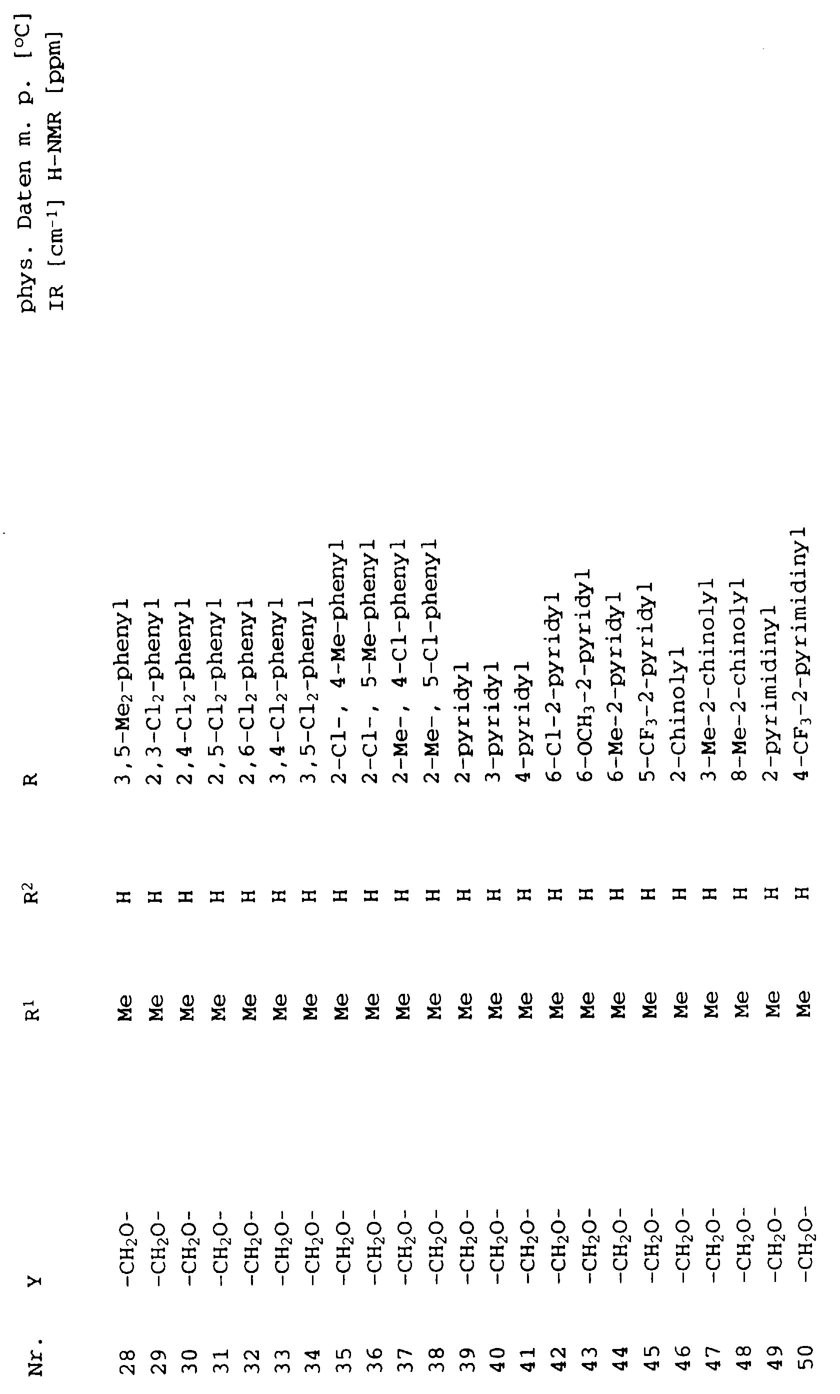

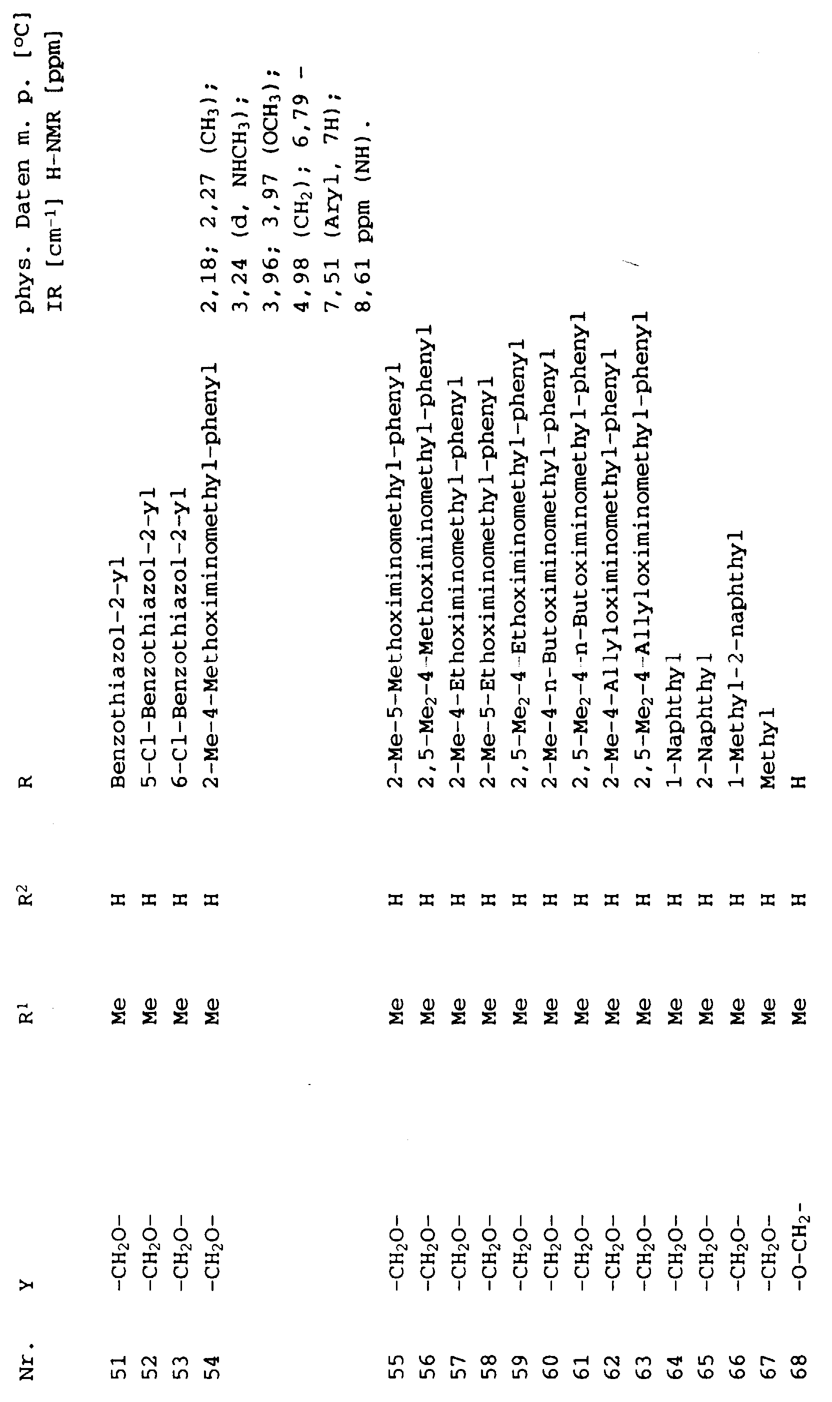

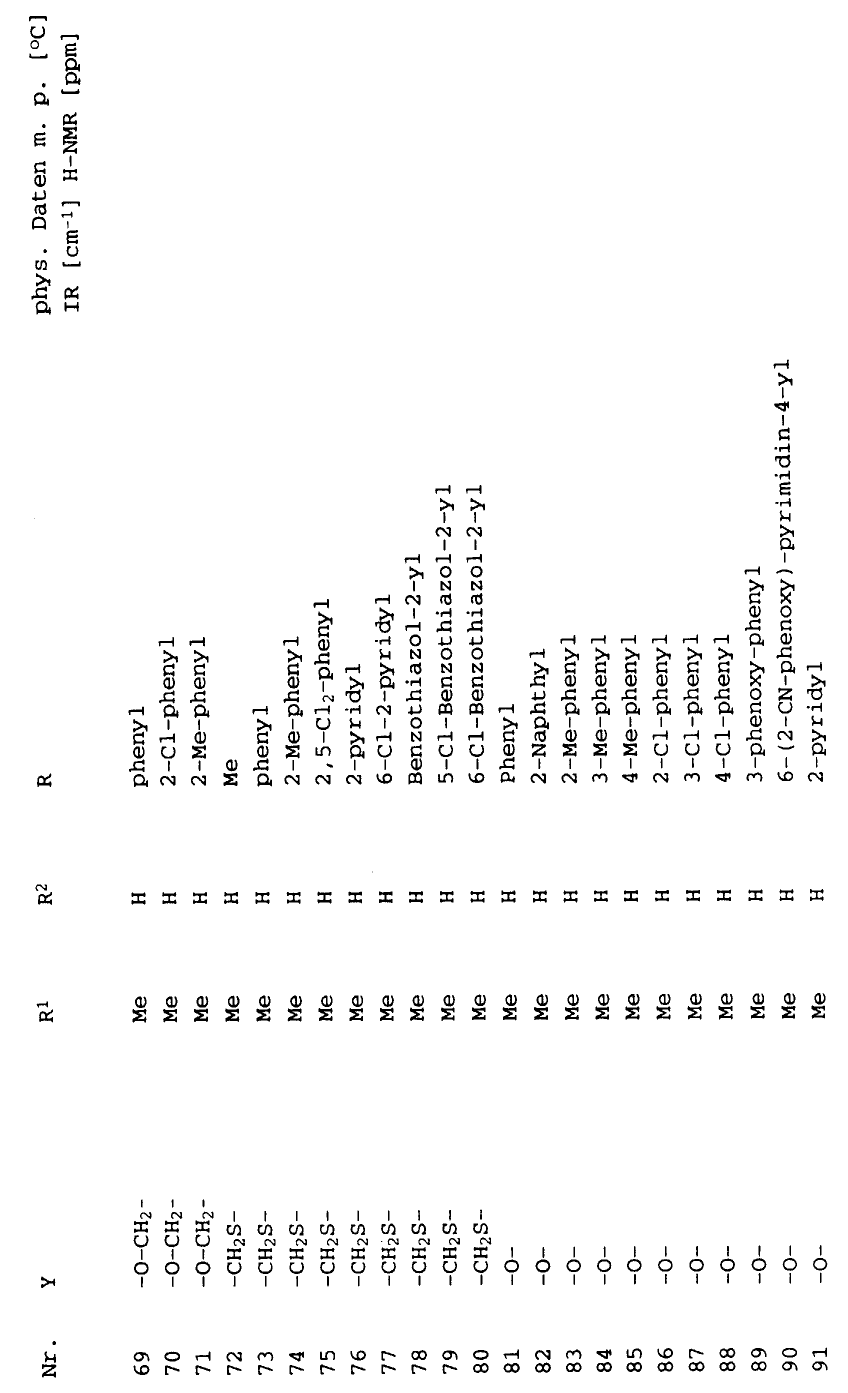

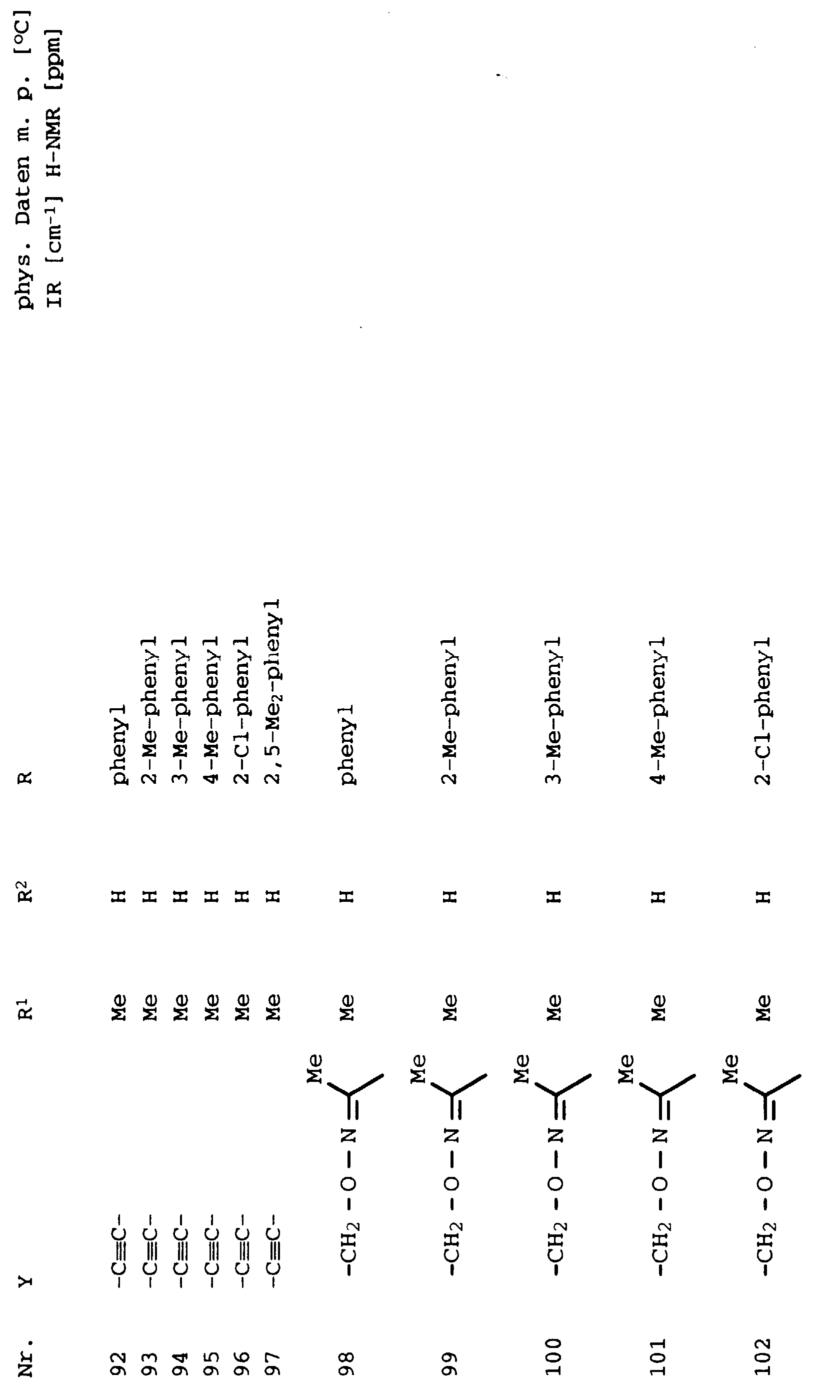

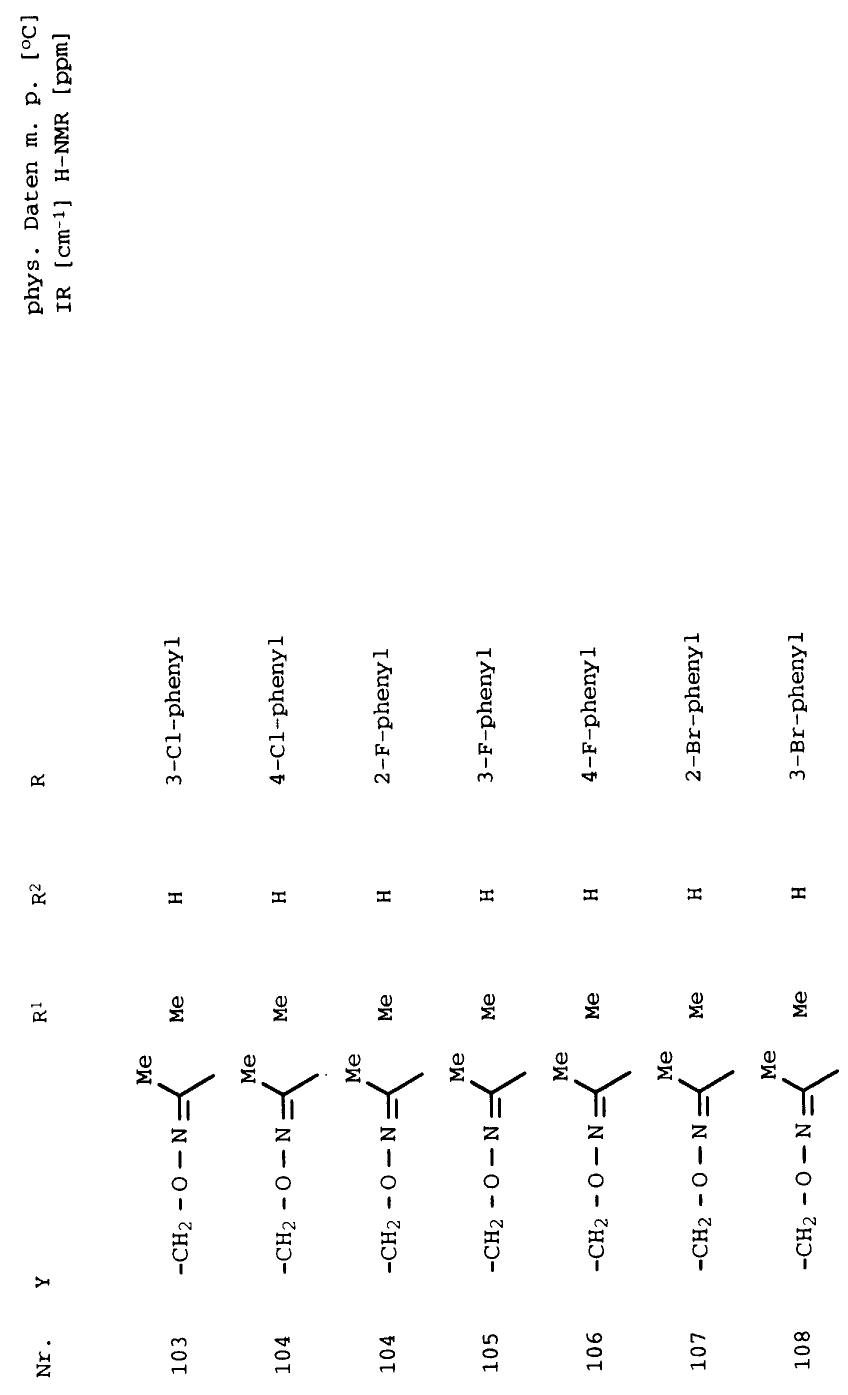

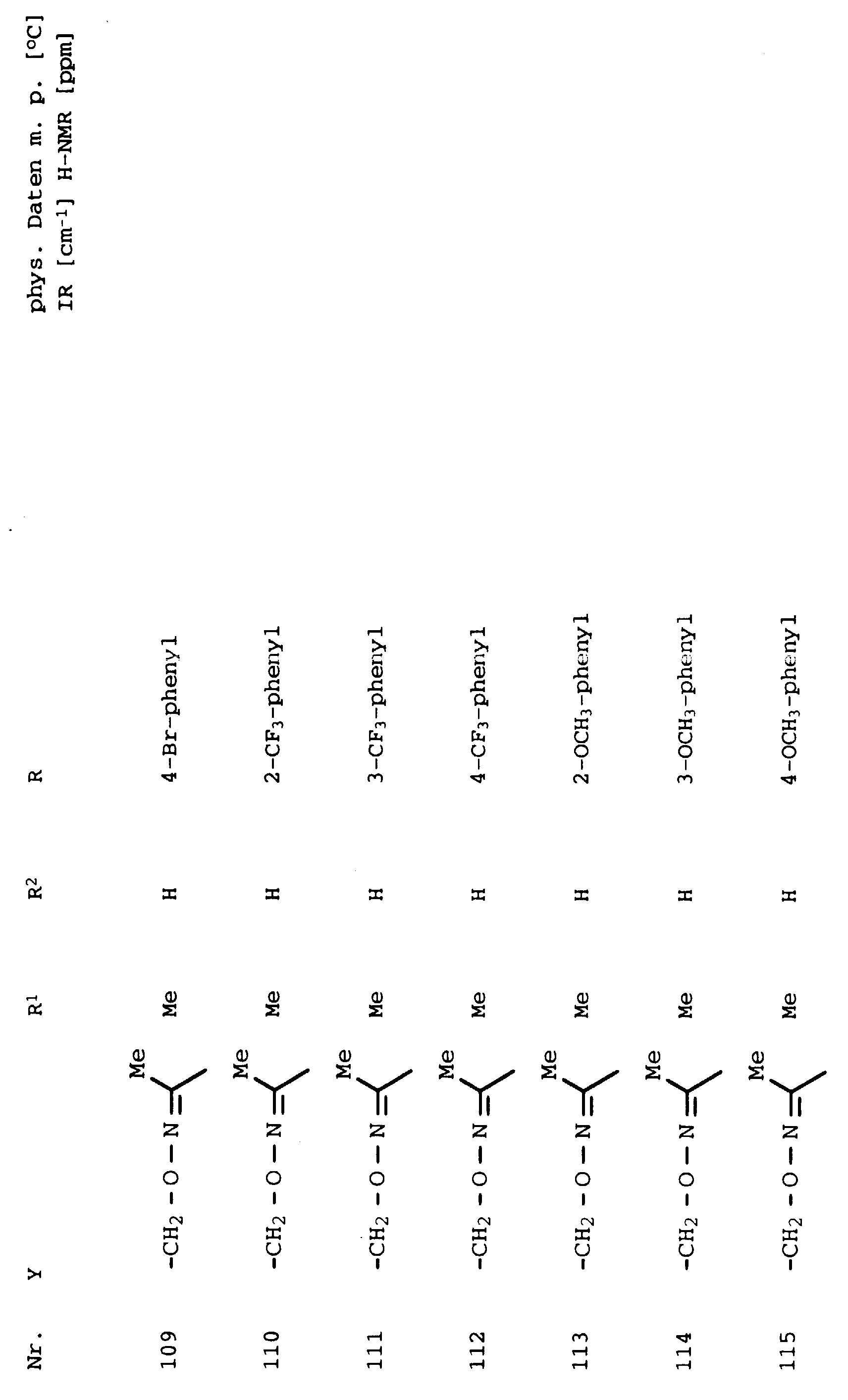

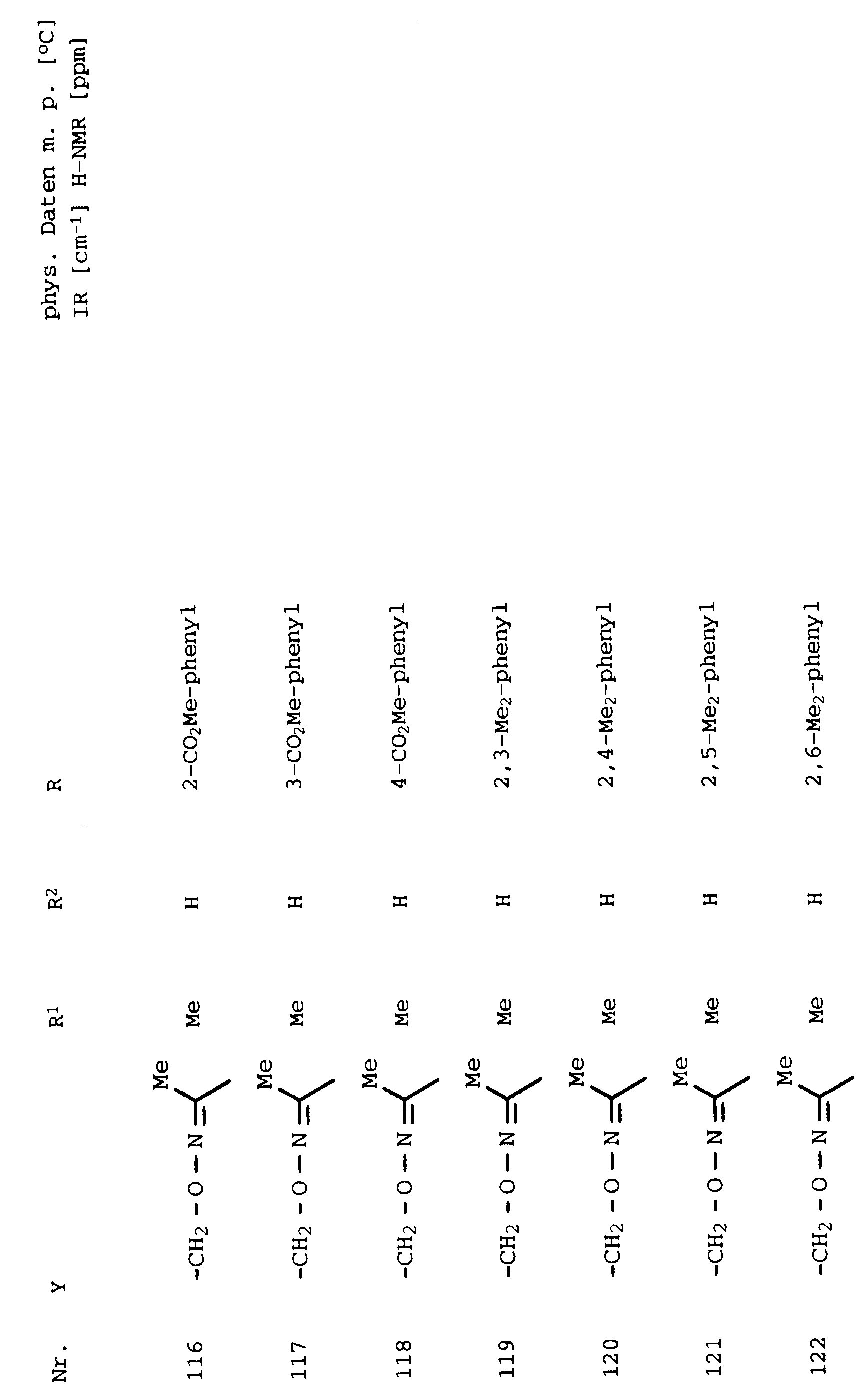

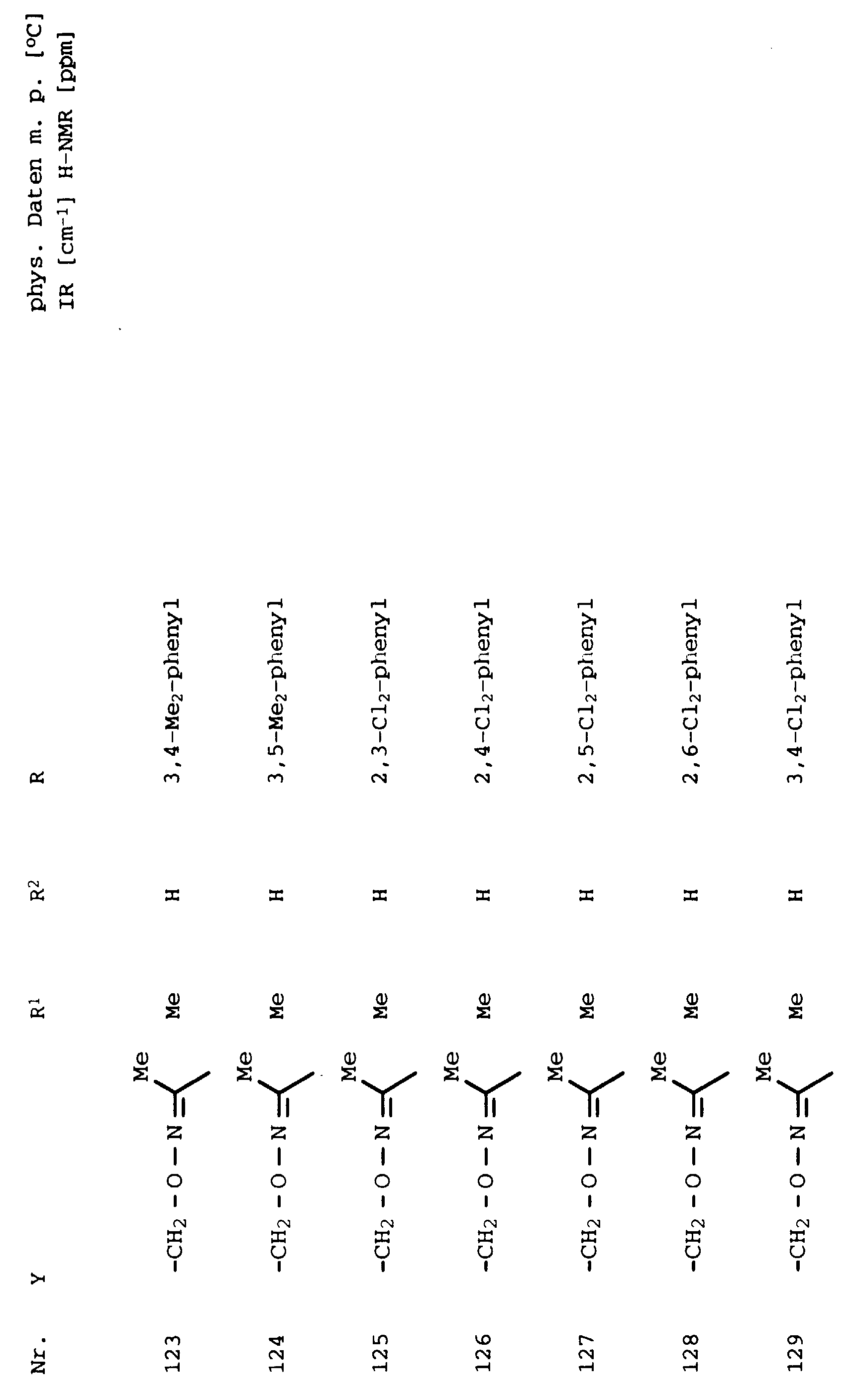

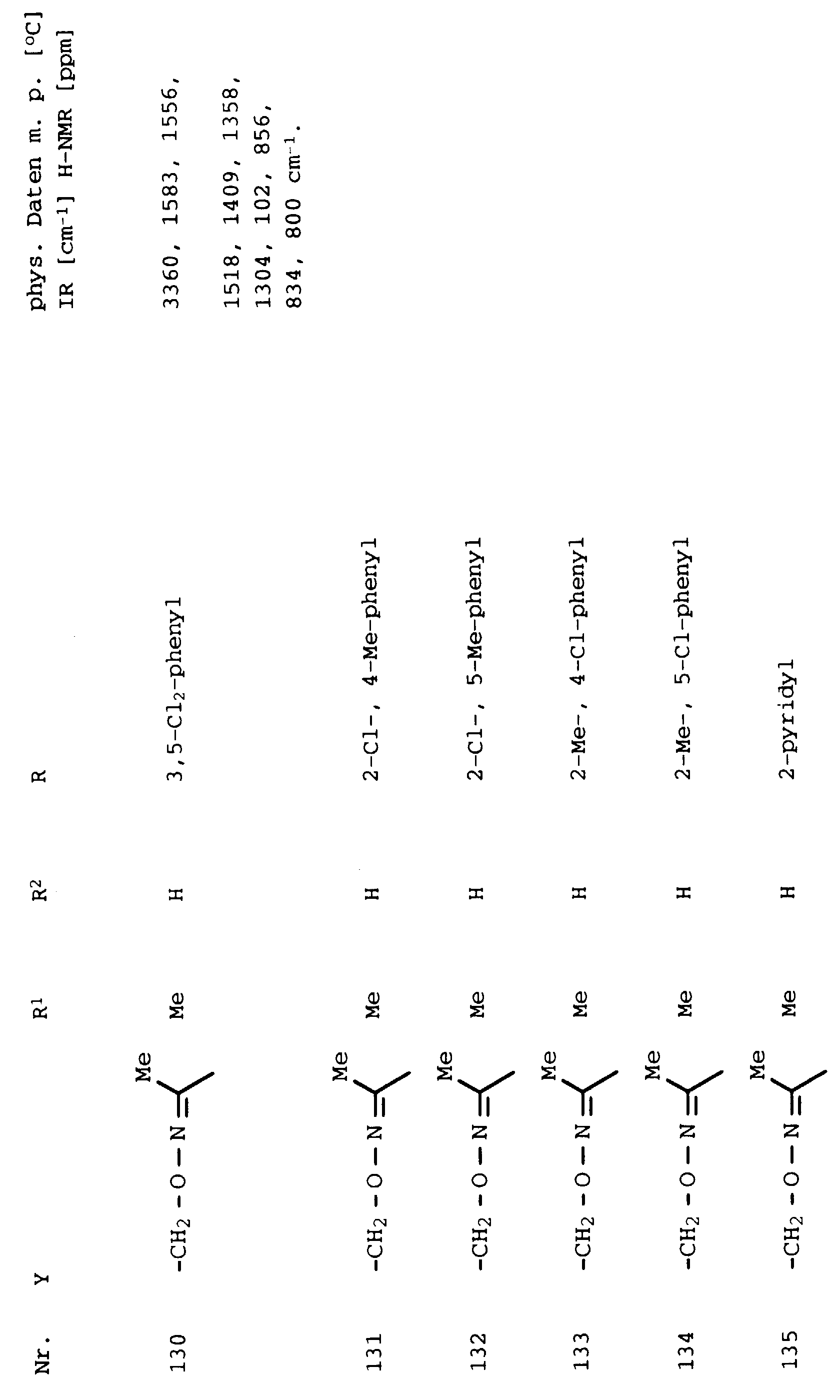

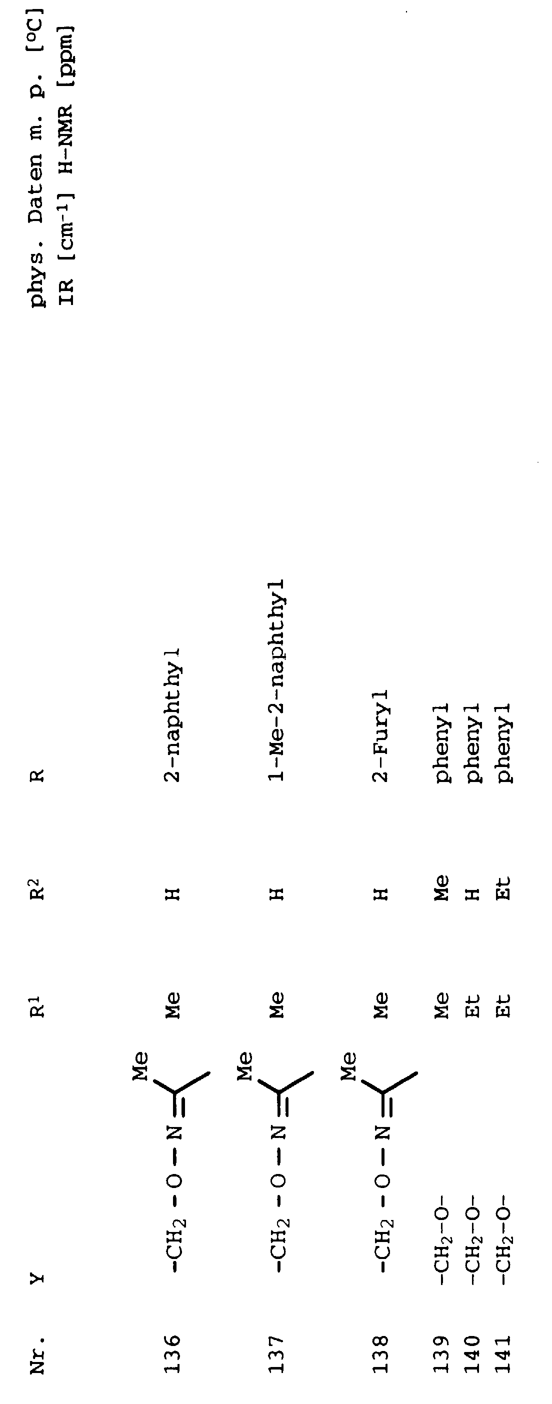

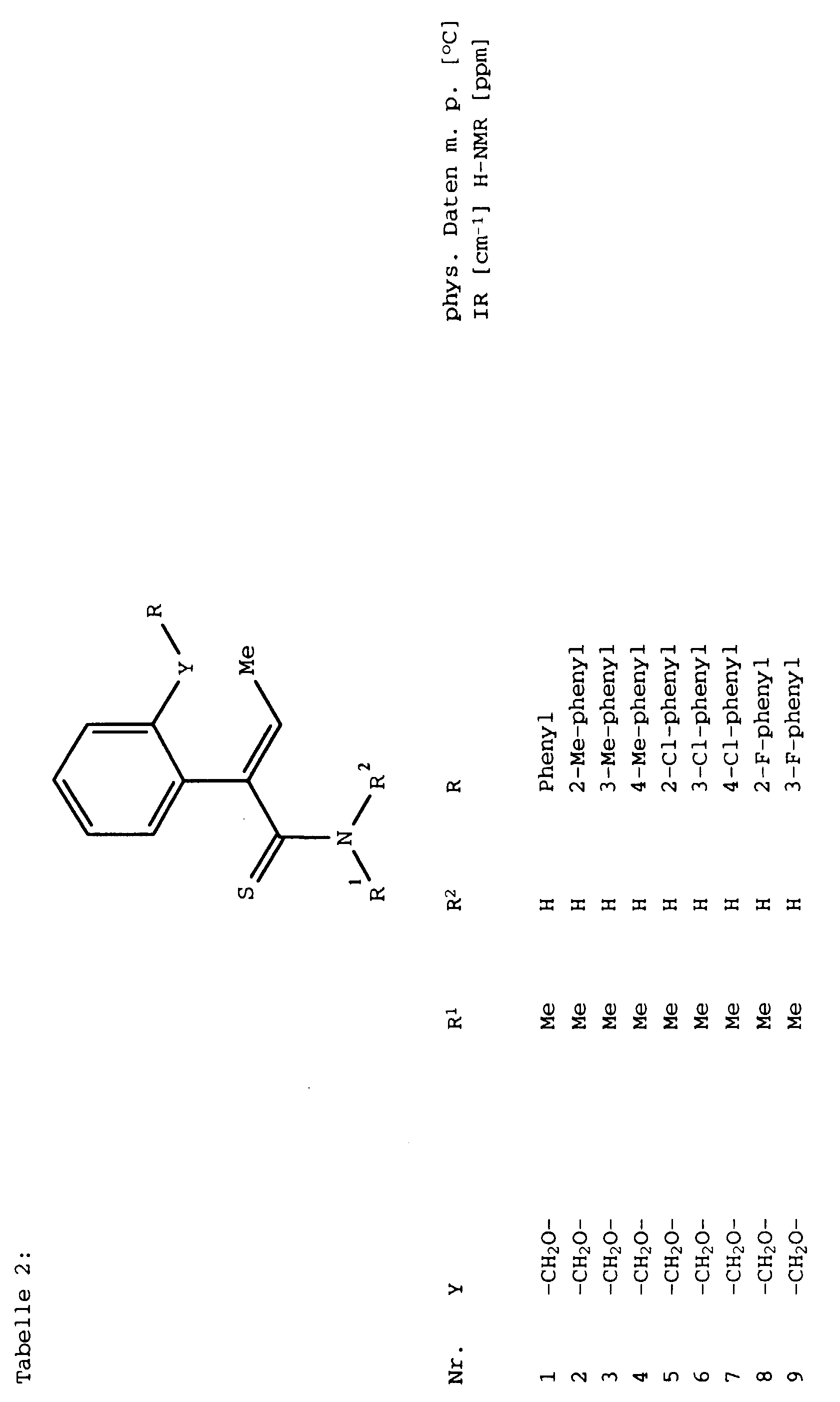

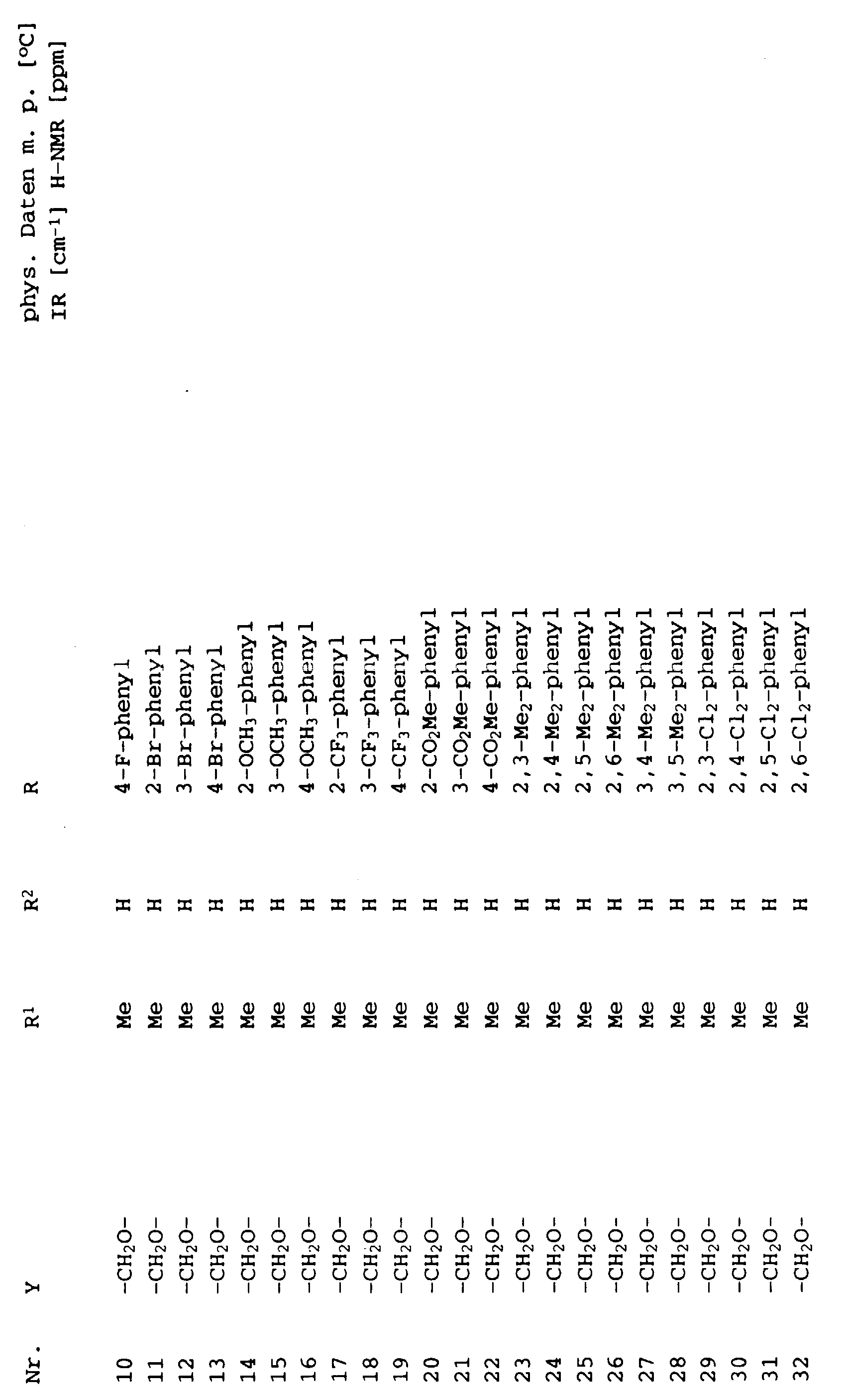

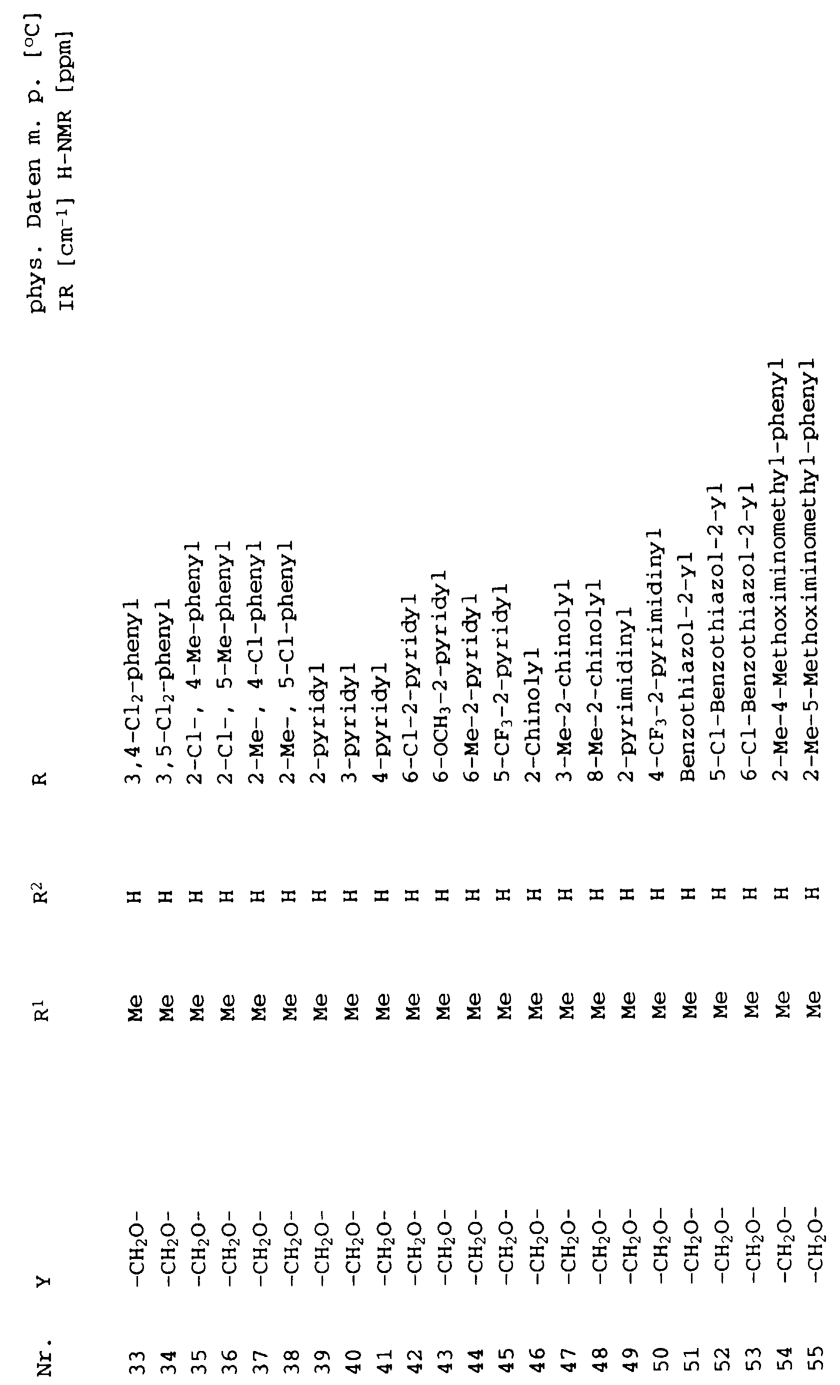

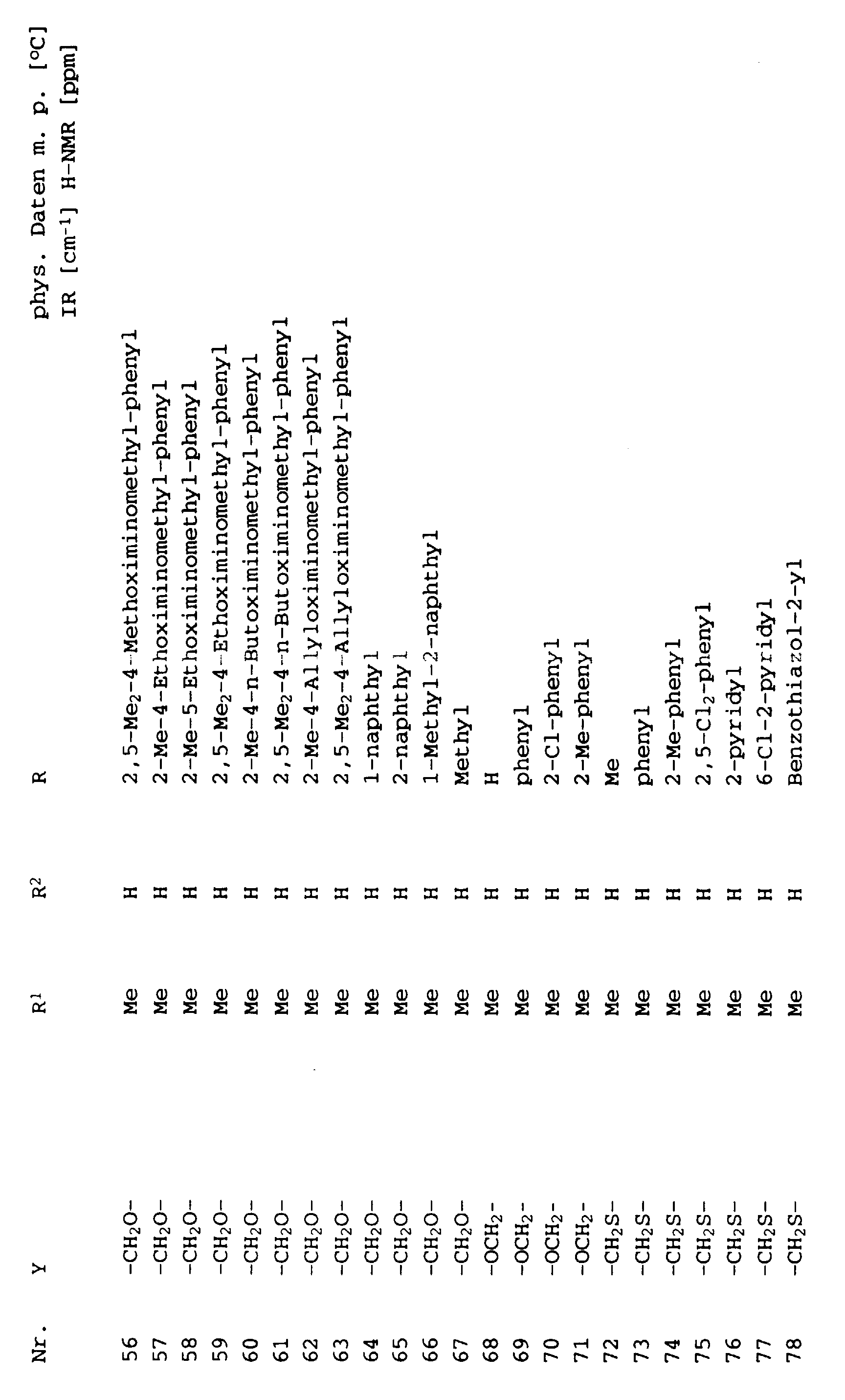

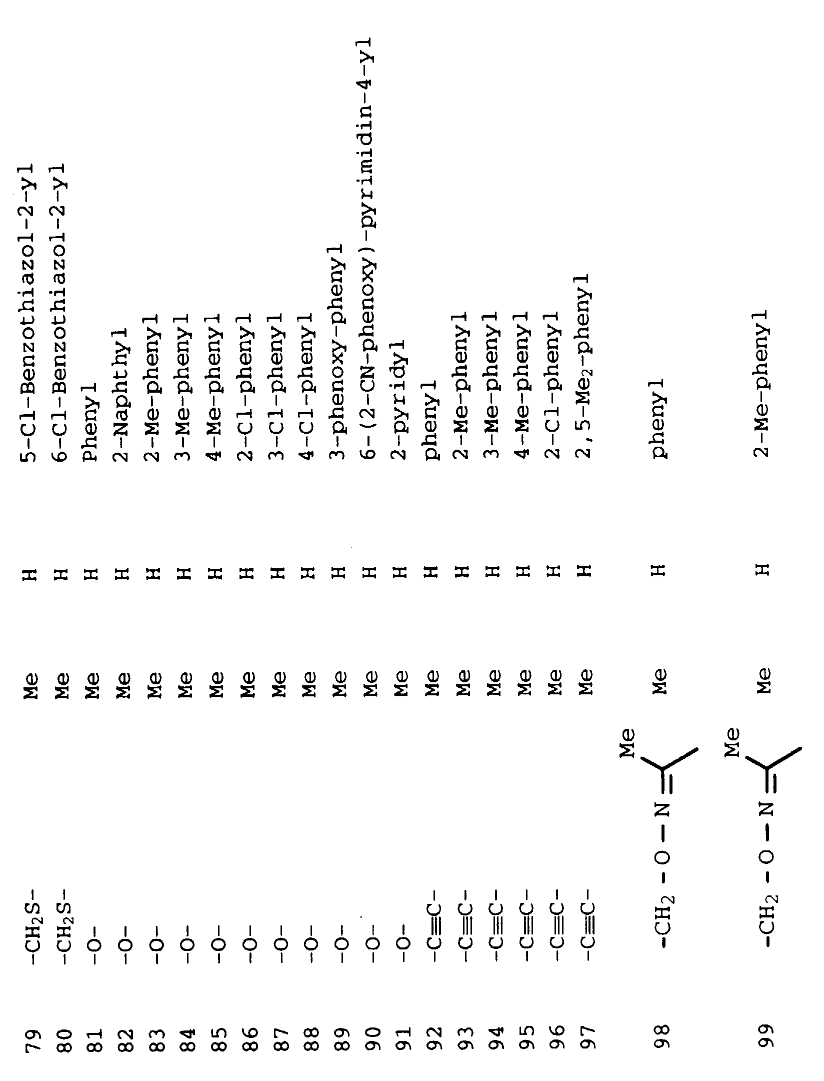

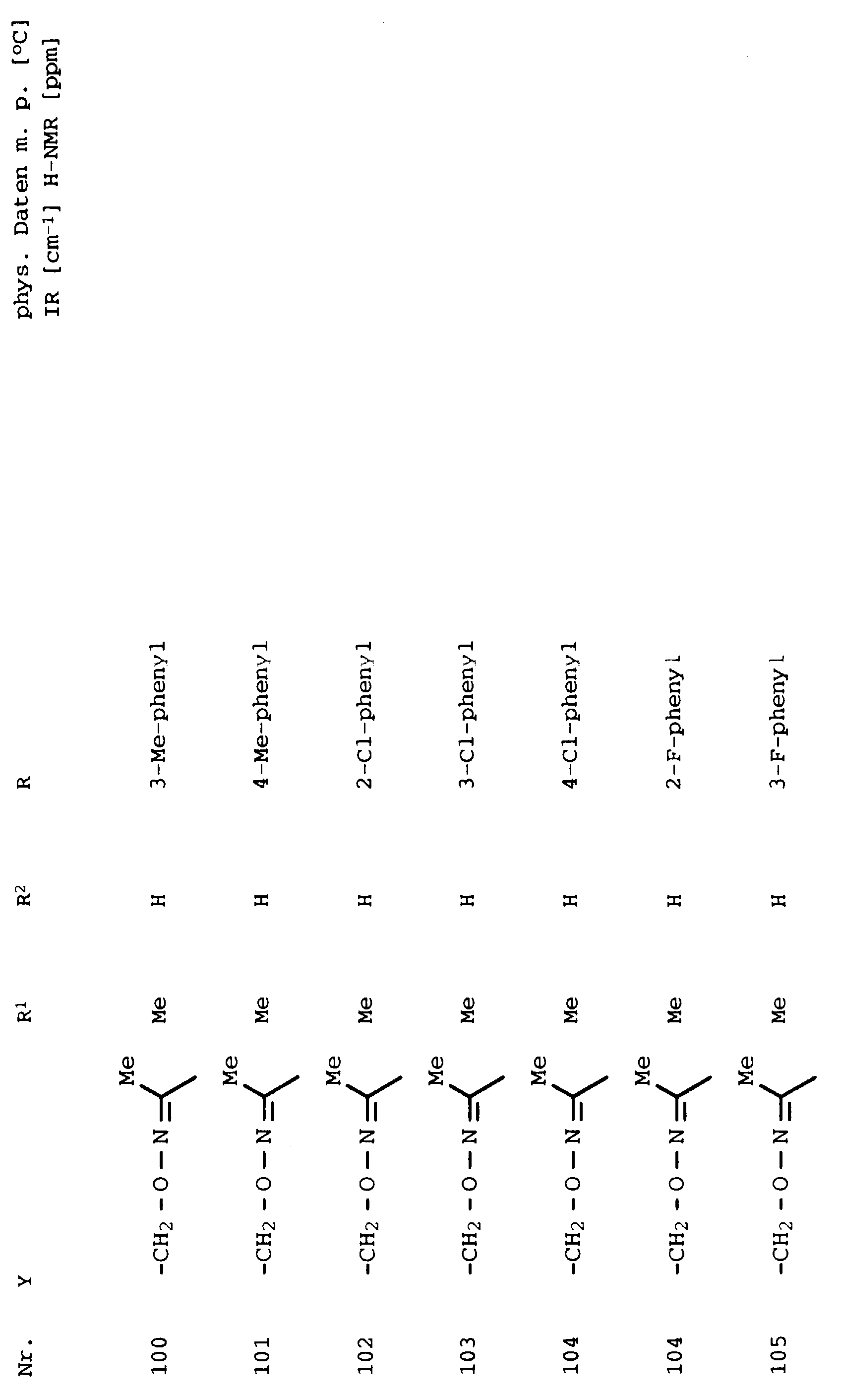

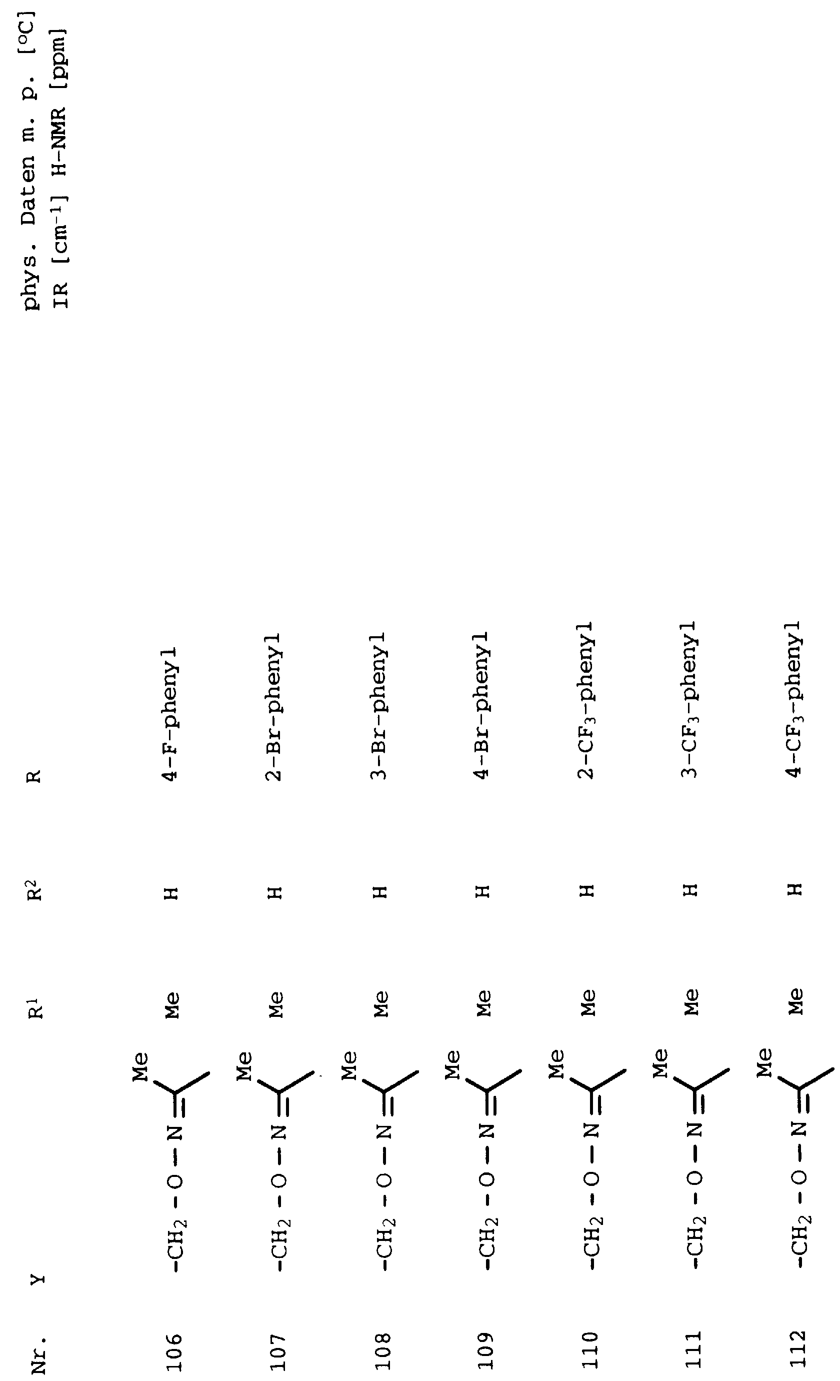

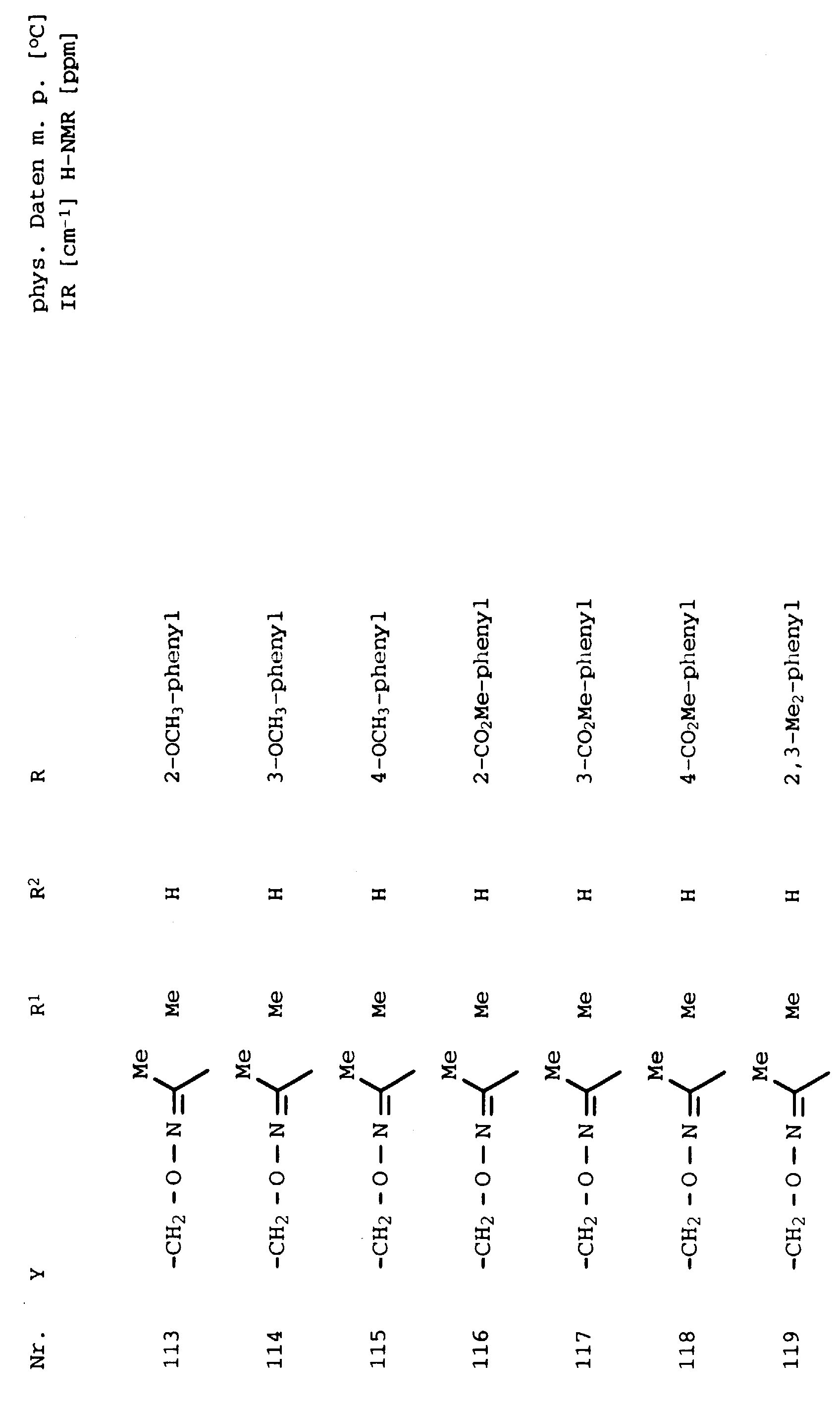

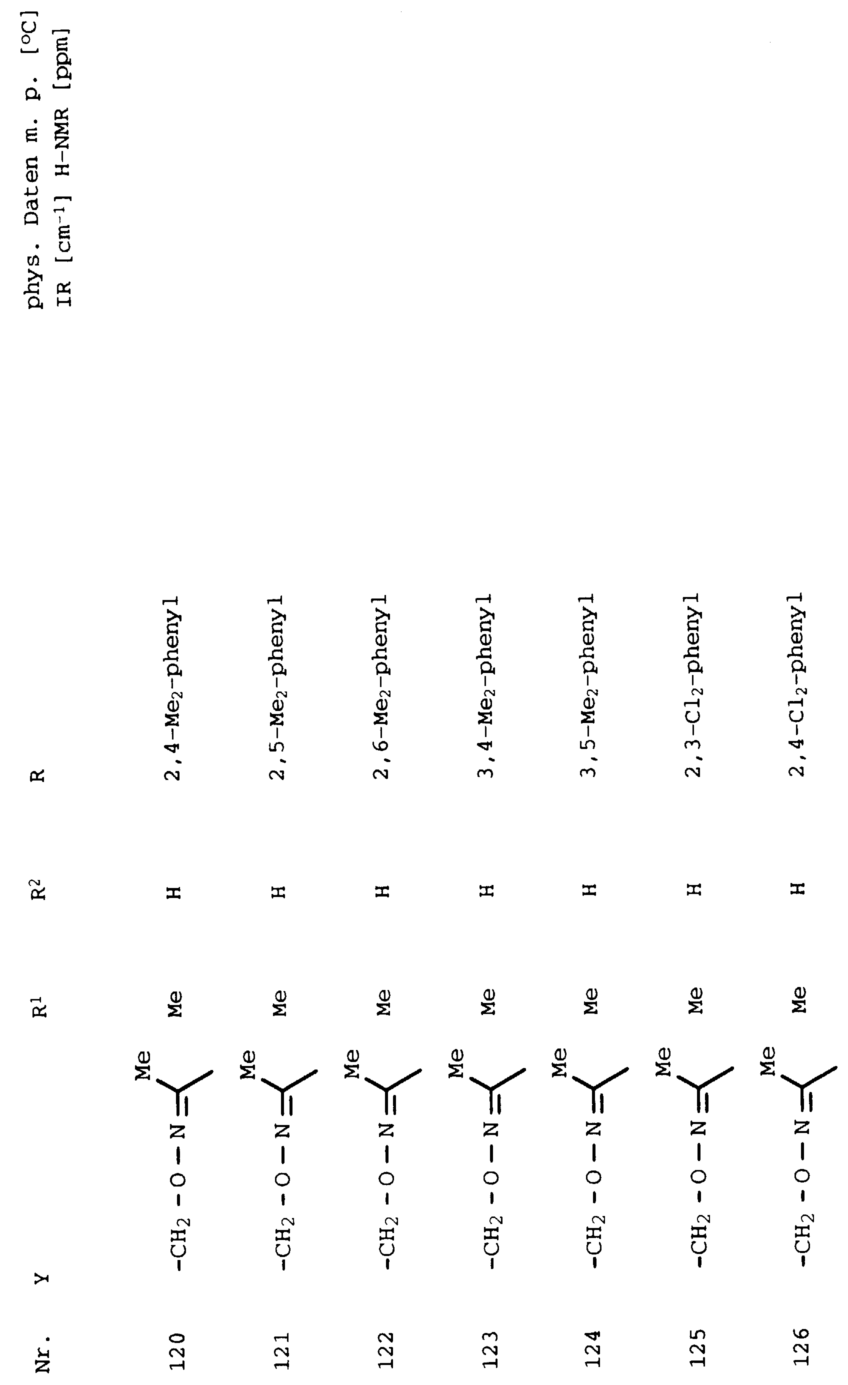

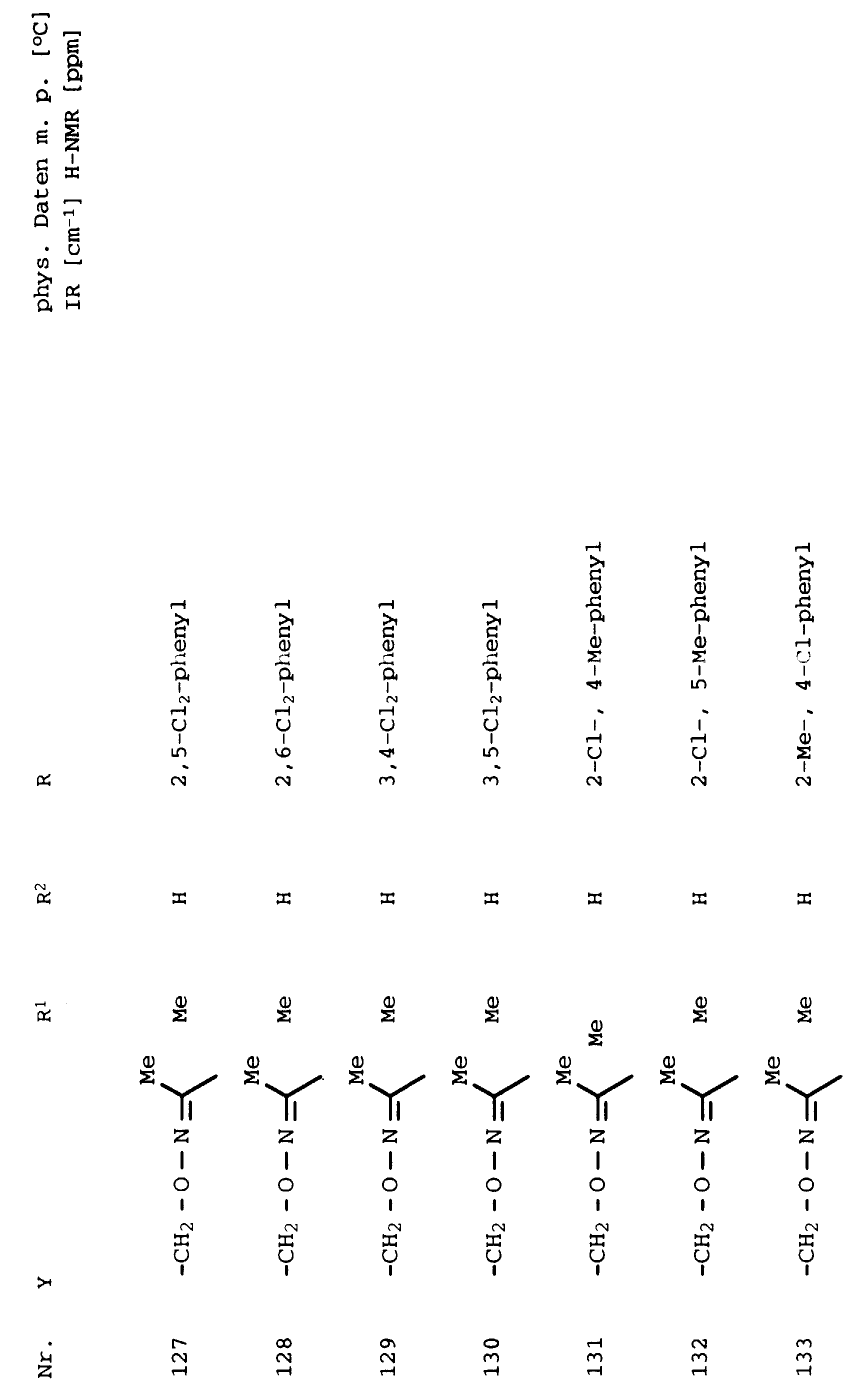

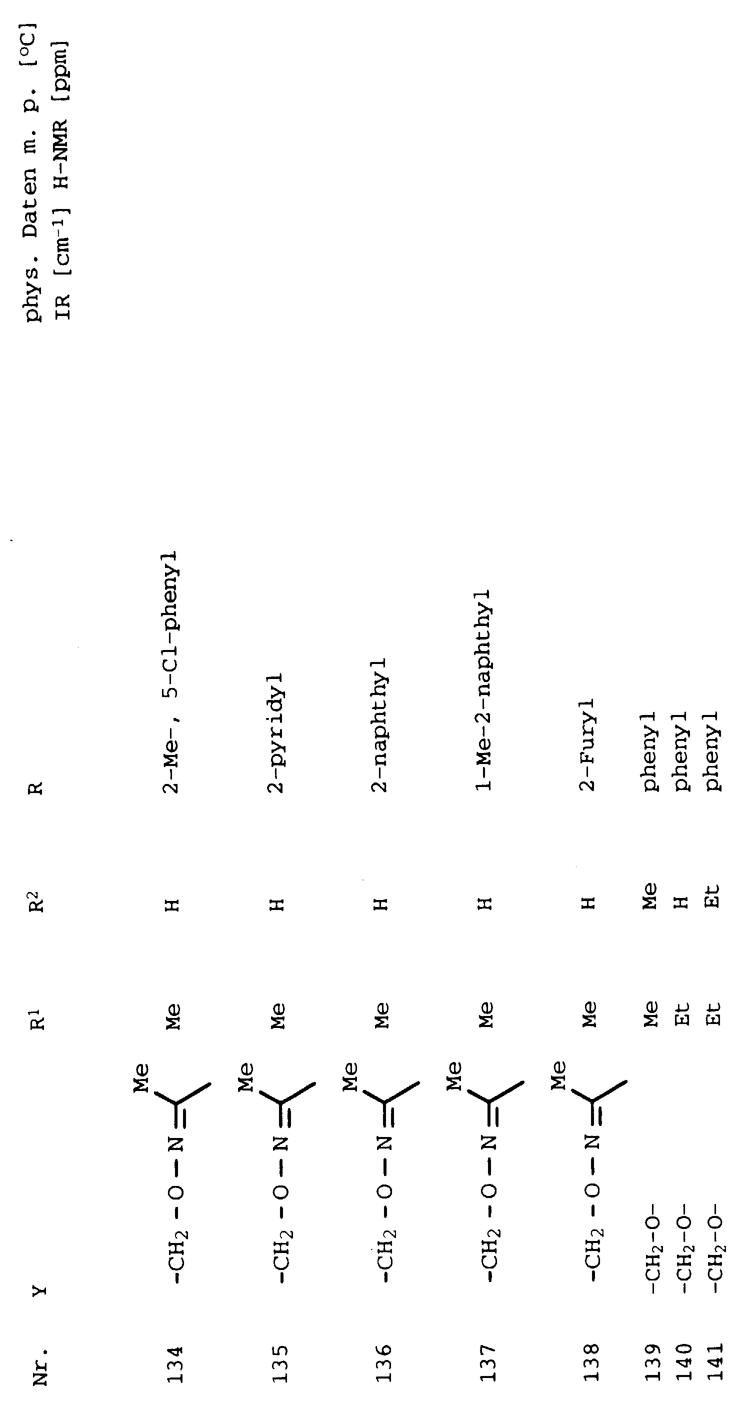

Die neuen Verbindungen eignen sich als Fungizide, Insektizide, Nematizide.

Die erfindungsgemäßen Wirkstoffe bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und und Fettalkoholsulfaten, sowie Salze sulfatierter Hexa-, Hepta- Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
- I.: eine Lösung aus 90 Gew.-Teilen der Verbindung 2 aus Tabelle 1 und 10 Gew.-Teilen N-Methyl-a-pyrrolidon, die zur Anwendung in Form kleinster Tropfen geeignet ist;
- II.: eine Mischung aus 20 Gew.-Teilen der Verbindung 25 aus Tabelle 1, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.
- III.: eine wäßrige Dispersion aus 20 Gew.-Teilen der Verbindung 54 aus Tabelle 1, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
- IV.: eine wäßrige Dispersion aus 20 Gew.-Teilen der Verbindung 130 aus Tabelle 1, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
- V.: eine in einer Hammermühle vermahlene Mischung aus 80 Gew.-Teilen der Verbindung 2 aus Tabelle 1, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
- VI.: eine innige Mischung aus 3 Gew.-Teilen der Verbindung 25 aus Tabelle 1 und 97 Gew.-Teilen feinteiligem Kaolin; dieses Stäubemittel enthält 3 Gew.-% Wirkstoff;
- VII.: eine innige Mischung aus 30 Gew.-Teilen der Verbindung 54 aus Tabelle 1, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
- VIII.: eine stabile wäßrige Dispersion aus 40 Gew.-Teilen der Verbindung 130 aus Tabelle 1, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
- IX.: eine stabile ölige Dispersion aus 20 Gew.-Teilen der Verbindung 2 aus Tabelle 1, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Wirkstoffe sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Die folgenden Anwendungsbeispiele zeigen die gute fungizide Wirkung der neuen Verbindungen.

### Anwendungsbeispiel 1

### Wirksamkeit gegen Plasmopara Viticola

Blätter von Tropfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Das Ergebnis des Versuchs zeigt, daß bei der Anwendung einer 63 ppm Wirkstoff enthaltenden Spritzbrühe die Verbindungen der Tabelle I, Nr. 2, 25 und 54 eine sehr gute fungizide Wirkung haben (0 % Befall).

### Anwendungsbeispiel 2

### Wirksamkeit gegen Botrytis cinera an Paprikaschoten

Scheiben von grünen Paprikaschoten wurden mit wäßriger Wirkstoffaufbereitung, die 80 % Wirkstoff und 20 % Emulgiermittel in der Trockensubstanz enthielt, tropfnaß besprüht. 2 Stunden nach dem Antrocknen des Spritzbelages wurden die Fruchtscheiben mit einer Sporensuspension von Botrytis cinera, die 1,7 x 10⁶ Sporen pro ml einer 2 %igen Biomalzlösung enthielt, inokuliert. Die inokulierten Fruchtscheiben wurden anschließend in feuchten Kammern bei 18°C für 4 Tage inkubiert. Dann erfolgte visuell die Auswertung der Botrytis-Entwicklung auf den befallenen Fruchtscheiben.

Das Ergebnis des Versuchs zeigt, daß bei der Anwendung einer 500 ppm Wirkstoff enthaltenden Spritzbrühe die Verbindungen der Tabelle I, Nr. 2, 25 und 54 eine sehr gute fungizide Wirkung haben (5 % Befall).

## Patentansprüche (Patentansprüche für folgende(n) Vertragsstaat(en): BE, CH, DE, DK, FR, GB, IE, IT, NL, SE, LI)

1. Thioamide der allgemeinen Formel I, 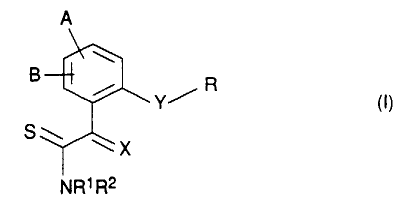 in der die Substituenten die folgende Bedeutung haben:
A, B, R' Wasserstoff, Cyano, C₁-C₄-Alkyl, C₁-C₄-Alkoxy oder Halogen,
R¹, R² Wasserstoff oder Methyl, und
X =CHCH₃ oder =N-OCH₃,
Y -O-, -S-, -SO-, -SO₂-, -OCH₂-, -CH₂O-, -SCH₂-, -CH₂S-, -CH₂CH₂-, -CH=CH-, -C≡C- und -CH₂O-N=CR'-, und
R Wasserstoff, ggf. subst. C₁-C₁₀-Alkyl, ggf. subst. C₃-C₆-Cycloalkyl, ggf. subst. Aryl oder ggf. subst. Hetaryl, oder
wobei der Ausdruck "ggf. subst." neben Wasserstoff auch Halogen, Cyano, Nitro, CO₂-(C₁-C₄-Alkyl) CO-(C₁-C₄-Alkyl), C₁-C₄-Alkyl, C₁-C₄-Alkoxy, C₁-C₄-Halogenalkyl, C₁-C₄-Alkoximino-C₁-C₄-alkyl, Aryl, Aryloxy, Benzyloxy, Hetaryl, Hetaryloxy, C₃-C₆-Cycloalkyl bedeutet, und
wobei der Ausdruck "Hetaryl" für einen ggf. subst. aromatischen, ein-, zwei- oder dreikernigen Fünfring- oder Sechsring-Heterocyclus steht.
ausgenommen Verbindungen, in denen A und B Wasserstoff bedeuten, R¹ Wasserstoff bedeutet und R² Wasserstoff oder Methyl bedeutet, wenn Y für -O- oder -CH₂O-steht und
R Phenyl bedeutet, welches unsubstituiert ist oder ein bis drei Halogenatome, oder ein oder zwei Methylgruppen oder eine der folgenden Gruppen trägt: Phenyl, Propinyloxy, Phenoxy oder Isopropyloxy, oder
R 2-Pyridyl bedeutet, welches unsubstituiert ist oder ein Halogenatom und eine C₁-Halogenalkygruppe trägt.

2. Verbindungen der Formel I gemäß Anspruch 1, in denen X für CHCH₃ steht, R¹, A und B Wasserstoff und R² Methyl bedeutet.

3. Verbindungen der Formel I gemäß Anspruch 1, in denen X für NOCH₃ steht, R¹, A und B Wasserstoff und R² Methyl bedeutet.

4. Verbindung der Formel I gemäß Anspruch 1, in der Y CH₂O, R¹ Methyl, A, B und R² Wasserstoff, R 4-Methoxyiminomethylphenyl und X NOCH₃ bedeuten.

5. Verbindungen der Formel I' 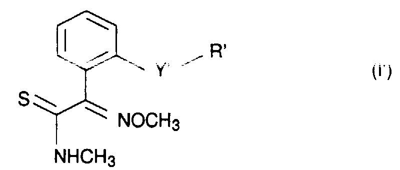 in denen die Gruppierung Y'-R' für 2-Pyridyl-oxy, 2-Pyridyloxymethylen, 2-(6-Chlorpyridyl)oxymethylen oder 2-(5-Trifluormethylpyridyl)oxymethylen steht.

6. Verfahren zur Herstellung von Verbindungen der Formel I gemäß Anspruch 1, dadurch gekennzeichnet, daß man ein Amid der Formel II 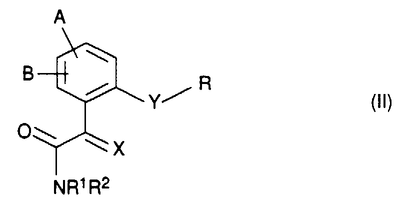 mit einem Schweflungsmittel umsetzt.

7. Schädlingsbekämpfungsmittel und Pilzbekämpfungsmittel, enthaltend einen inerten Trägerstoff und ein Thioamidderivat der Formel I gemäß Anspruch 1 oder ein Thioamidderivat der Formel I' gemäß Anspruch 5.

8. Verfahren zur Bekämpfung von Pilzen, dadurch gekennzeichnet, daß man die Pilze oder die von Pilzbefall bedrohten Materialien, Pflanzen, Saatgut oder den Erdboden mit einer fungizid wirksamen Menge einer Verbindung der Formel I gemäß Anspruch 1 oder einem Thioamidderivat der Formel I' gemäß Anspruch 5 behandelt.

9. Verfahren zur Bekämpfung von Schädlingen, dadurch gekennzeichnet, daß man eine insektizid, nematizid und/oder akarizid wirksame Menge eines Thioamids der Formel I gemäß Anspruch 1 oder eines Thioamidderivats der Formel I' gemäß Anspruch 5 auf Insekten, Nematoden oder Spinnmilben oder auf deren Lebensraum einwirken läßt.

## Patentansprüche (Patentansprüche für folgende(n) Vertragsstaat(en): AT, ES, GR, PT)

1. Thioamide der allgemeinen Formel I, 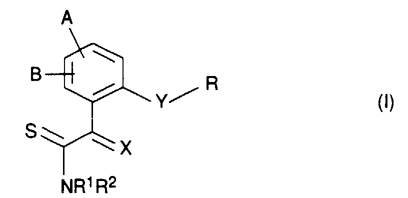 in der die Substituenten die folgende Bedeutung haben:
A, B, R' Wasserstoff, Cyano, C₁-C₄-Alkyl, C₁-C₄-Alkoxy oder Halogen,
X =CHCH₃ oder =N-OCH₃,
R¹, R² Wasserstoff oder C₁-C₄-Alkyl,
Y -O-, -S-, -SO-, -SO₂-, -OCH₂-, -CH₂O-, -SCH₂-, -CH₂S-, -CH₂CH₂-, -CH=CH-, -C≡C- und -CH₂O-N=CR'-, und
R Wasserstoff, ggf. subst. C₁-C₁₀-Alkyl, ggf. subst. C₃-C₆-Cycloalkyl, ggf. subst. Aryl oder ggf. subst. Hetaryl bedeutet,
wobei der Ausdruck "ggf. subst." neben Wasserstoff auch Halogen, Cyano, Nitro, CO₂-(C₁-C₄-Alkyl) CO-(C₁-C₄-Alkyl), C₁-C₄-Alkyl, C₁-C₄-Alkoxy, C₁-C₄-Halogenalkyl, C₁-C₄-Alkoximino-C₁-C₄-alkyl, Aryl, Aryloxy, Benzyloxy, Hetaryl, Hetaryloxy, C₃-C₆-Cycloalkyl bedeutet, und
wobei der Ausdruck "Hetaryl" für einen ggf. subst. aromatischen, ein-, zwei- oder dreikernigen Fünfringoder Sechsring-Heterocyclus steht.

2. Verbindungen der Formel I gemäß Anspruch 1, in denen X für CHCH₃ steht, R¹, A und B Wasserstoff und R² Methyl bedeutet.

3. Verbindungen der Formel I gemäß Anspruch 1, in denen X für NOCH₃ steht, R¹, A und B Wasserstoff und R² Methyl bedeutet.

4. Verfahren zur Herstellung von Verbindungen der Formel I gemäß Anspruch 1, dadurch gekennzeichnet, daß man ein Amid der Formel II 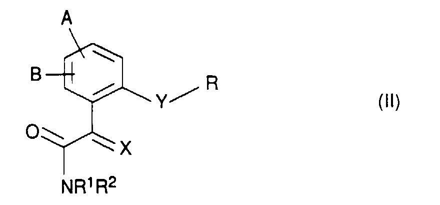 mit einem Schweflungsmittel umsetzt.

5. Schädlingsbekämpfungsmittel und Pilzbekämpfungsmittel, enthaltend einen inerten Trägerstoff und ein Thioamidderivat der Formel I gemäß Anspruch 1.

6. Verfahren zur Bekämpfung von Pilzen, dadurch gekennzeichnet, daß man die Pilze oder die von Pilzbefall bedrohten Materialien, Pflanzen, Saatgut oder den Erdboden mit einer fungizid wirksamen Menge einer Verbindung der Formel I gemäß Anspruch 1 behandelt.

7. Verfahren zur Bekämpfung von Schädlingen, dadurch gekennzeichnet, daß man eine insektizid, nematizid und/oder akarizid wirksame Menge eines Thioamids der Formel I gemäß Anspruch 1 auf Insekten, Nematoden oder Spinnmilben oder auf deren Lebensraum einwirken läßt.

8. Verbindung der Formel I gemäß Anspruch 1, in der Y CH₂O, R¹ Methyl, A, B und R² Wasserstoff, R 2-Methylphenyl und X NOCH₃ bedeuten.

9. Verbindung der Formel I gemäß Anspruch 1, in der Y CH₂O, R¹ Methyl, A, B und R² Wasserstoff, R 2,5-Dimethylphenyl und X NOCH₃ bedeuten.

10. Verbindung der Formel I gemäß Anspruch 1, in der Y CH₂O, R¹ Methyl, A, B und R² Wasserstoff, R 4-Methoxyiminomethylphenyl und X NOCH₃ bedeuten.

## Claims (Claims for the following Contracting State(s): BE, CH, DE, DK, FR, GB, IE, IT, NL, SE, LI)

1. Thioamides of the general formula I 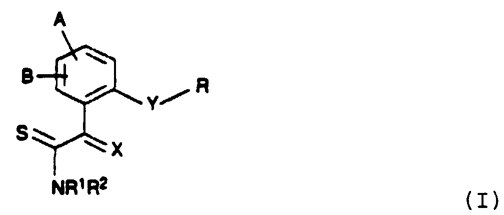 where:
A, B and R' are each hydrogen, cyano, C₁-C₄-alkyl, C₁-C₄-alkoxy or halogen,
R¹ and R² are each hydrogen or methyl, and
X is =CHCH₃ or =N-OCH₃,
Y is -O-, -S- , -SO-, -SO₂- , -OCH₂- , -CH₂O-, -SCH₂-, -CH₂S- , -CH₂CH₂- , -CH=CH-, -C=C- and -CH₂O-N=CR'- , and
R is hydrogen, opt. subst. C₁-C₁₀-alkyl, opt. subst. C₃-C₆-cycloalkyl, opt. subst. aryl or opt. subst. hetaryl,
where the term "opt. subst." refers, in addition to hydrogen, also to halogen, cyano, nitro, CO₂-(C₁-C₄-alkyl), CO-(C₁-C₄-alkyl), C₁-C₄-alkyl, C₁-C₄-alkoxy, C₁-C₄-haloalkyl, C₁-C₄-alkoximino-C₁-C₄-alkyl, aryl, aryloxy, benzyloxy, hetaryl, hetaryloxy and C₃-C₆-cycloalkyl, and
where the term "hetaryl" is an opt. subst. aromatic, mono-, di- or trinuclear five ring or six ring heterocycle,
excluding compounds where A and B are each hydrogen, R¹ is hydrogen and R² is hydrogen or methyl, if Y is -O- or -CH₂O- and
R is unsubstituted phenyl or phenyl carrying one to three halogens or one or two methyl groups or one of the following groups: phenyl, propynyloxy, phenoxy or isopropyloxy, or
R is unsubstituted 2-pyridyl or 2-pyridyl carrying a halogen and a C₁-haloalkyl group.

2. Compounds of the formula I as claimed in claim 1 where X is CHCH₃, R¹, A and B are each hydrogen and R² is methyl.

3. Compounds of the formula I as claimed in claim 1 where X is NOCH₃, R¹, A and B are each hydrogen and R² is methyl.

4. Compound of the formula I as claimed in claim 1 where Y is CH₂O, R¹ is methyl, A, B and R² are each hydrogen, R is 4-methoxyiminomethylphenyl and X is NOCH₃.

5. Compounds of the formula I' 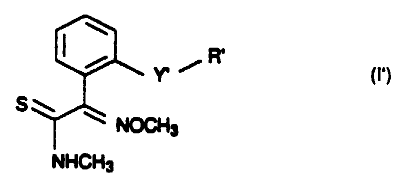 where the group Y'-R' is 2-pyridyloxy, 2-pyridyloxymethylene, 2-(6-chloropyridyl)oxymethylene or 2-(5-trifluoromethylpyridyl)oxymethylene.

6. A process for preparing compounds of the formula I as claimed in claim 1, characterized in that it comprises reacting an amide of the formula II 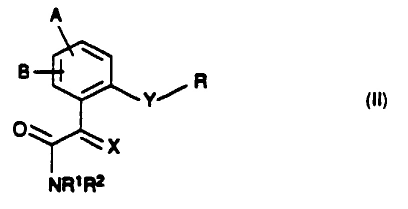 with a sulfurizing agent.

7. Pesticides and fungicides containing an inert carrier and a thioamide derivative of the formula I as claimed in claim 1 or a thioamide derivative of the formula I' as claimed in claim 5.

8. A method for controlling fungi, characterized in that it comprises treating the fungi or the materials, plants, seeds or the soil threatened by fungi with a fungicidally active amount of a compound of the formula I as claimed in claim 1 or a thioamide derivative of the formula I' as claimed in claim 5.

9. A method for controlling pests, characterized in that it comprises allowing an insecticidally, nematicidally and/or acaricidally active amount of a thioamide of the formula I as claimed in claim 1 or of a thioamide derivative of the formula I' as claimed in claim 5 to act on insects, nematodes or arachnids or on their habitat.

## Claims (Claims for the following Contracting State(s): AT, ES, GR, PT)

1. Thioamides of the general formula I 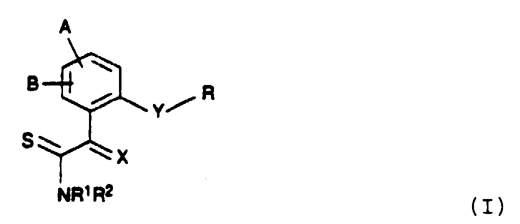 where:
A, B and R' are each hydrogen, cyano, C₁-C₄-alkyl, C₁-C₄-alkoxy or halogen,
X is =CHCH₃ or =N-OCH₃,
R¹ and R² are each hydrogen or C₁-C₄-alkyl,
Y is -O-, -S-, -SO-, -SO₂-, -OCH₂-, -CH₂O-, -SCH₂-, -CH₂S-, -CH₂CH₂-, -CH=CH-, -C=C- and -CH₂O-N=CR'-, and
R is hydrogen, opt. subst. C₁-C₁₀-alkyl, opt. subst. C₃-C₆-cycloalkyl, opt. subst. aryl or opt. subst. hetaryl,
where the term "opt. subst." refers, in addition to hydrogen, also to halogen, cyano, nitro, CO₂-(C₁-C₄-alkyl), CO-(C₁-C₄-alkyl), C₁-C₄-alkyl, C₁-C₄-alkoxy, C₁-C₄-haloalkyl, C₁-C₄-alkoximino-C₁-C₄-alkyl, aryl, aryloxy, benzyloxy, hetaryl, hetaryloxy and C₃-C₆-cycloalkyl, and
where the term "hetaryl" is an opt. subst. aromatic, mono-, di- or trinuclear five ring or six ring heterocycle.

2. Compounds of the formula I as claimed in claim 1 where X is CHCH₃, R¹, A and B are each hydrogen and R² is methyl.

3. Compounds of the formula I as claimed in claim 1 where X is NOCH₃, R¹, A and B are each hydrogen and R² is methyl.

4. A process for preparing compounds of the formula I as claimed in claim 1, characterized in that it comprises reacting an amide of the formula II 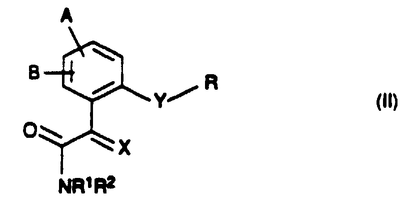 with a sulfurizing agent.

5. Pesticides and fungicides containing an inert carrier and a thioamide derivative of the formula I as claimed in claim 1.

6. A method for controlling fungi, characterized in that it comprises treating the fungi or the materials, plants, seeds or the soil threatened by fungi with a fungicidally active amount of a compound of the formula I as claimed in claim 1.

7. A method for controlling pests, characterized in that it comprises allowing an insecticidally, nematicidally and/or acaricidally active amount of a thioamide of the formula I as claimed in claim 1 to act on insects, nematodes or arachnids or on their habitat.

8. A compound of the formula I as claimed in claim 1 where Y is CH₂O, R¹ is methyl, A, B and R² are each hydrogen, R is 2-methylphenyl and X is NOCH₃.

9. A compound of the formula I as claimed in claim 1 where Y is CH₂O, R¹ is methyl, A, B and R² are each hydrogen, R is 2,5-dimethylphenyl and X is NOCH₃.

10. A compound of the formula I as claimed in claim 1 where Y is CH₂O, R¹ is methyl, A, B and R² are each hydrogen, R is 4-methoxyiminomethylphenyl and X is NOCH₃.

## Revendications (Revendications pour l'(les) Etat(s) contractant(s) suivant(s): BE, CH, DE, DK, FR, GB, IE, IT, NL, SE, LI)

1. Thioamides de formule générale I, 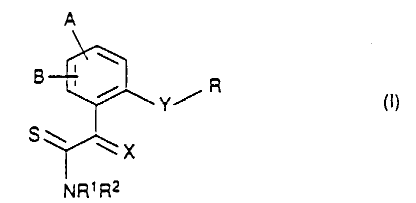 où les substituants ont la signification suivante:
A, B, R' hydrogène, cyano, alkyle en C₁-C₄, alcoxy en C₁-C₄ ou halogène,
R¹, R² hydrogène ou méthyle, et
X =CHCH₃ ou =N-OCH₃,
Y -O-, -S-, -SO-, -SO₂-, -OCH₂-, -CH₂O-, -SCH₂-, -CH₂S-, -CH₂CH₂-, -CH=CH-, -C≡C- et -CH₂O-N=CR'-, et
R hydrogène, alkyle en C₁-C₁₀ éventuellement substitué, cycloalkyle en C₃-C₆ éventuellement substitué, aryle éventuellement substitué ou hétéroaryle éventuellement substitué, ou
tandis que l'expression "éventuellement substitué" désigne outre l'hydrogène également un reste halogéno, cyano, nitro, CO₂-(alkyle en C₁-C₄), CO-(alkyle en C₁-C₄), alkyle en C₁-C₄, alcoxy en C₁-C₄, halogénoalkyle en C₁-C₄, alcoxyimino(C₁-C₄)-alkyle en C₁-C₄, aryle, aryloxy, benzyloxy, hétéroaryle, hétéroaryloxy, cycloalkyle en C₃-C₆, et
tandis que l'expression "hétéroaryle" désigne un hétérocycle aromatique, éventuellement substitué, à un, deux ou trois noyaux, penta- ou hexagonal,
à l'exception des composés dans lesquels
A et B représentent l'hydrogène, R¹ désigne l'hydrogène et R² désigne l'hydrogène ou un groupe méthyle, lorsque Y représente -O- ou -CH₂O-, et
R représente un reste phényle, qui est non-substitué ou porte un à trois atomes d'halogène, ou un ou deux groupes méthyle ou l'un des groupes suivants: phényle, propynyloxy, phénoxy ou isopropyloxy, ou
R représente un reste 2-pyridyle, qui est non-substitué ou porte un atome d'halogène et un groupe halogénoalkyle en C₁.

2. Composés de formule I selon la revendication 1, dans lesquels X représente CHCH₃, R¹, A et B représentent l'hydrogène et R² représente un groupe méthyle.

3. Composés de formule I selon la revendication 1, dans lesquels X représente NOCH₃, R¹, A et B représentent l'hydrogène et R² représente un groupe méthyle.

4. Composé de formule I selon la revendication 1, dans lequel Y représente CH₂O, R¹ représente un groupe méthyle, A, B et R² représentent l'hydrogène, R représente un groupe 4-méthoxyiminométhylphényle et X représente NOCH₃.

5. Composés de formule I' 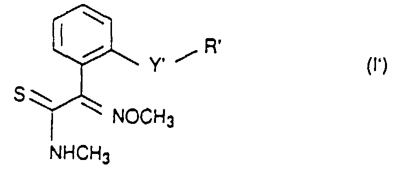 où le groupement Y'-R' représente un groupe 2-pyridyloxy, 2-pyridyloxyméthylène, 2-(6-chloropyridyl)oxyméthylène ou 2-(5-trifluorométhylpyridyl)oxyméthylène.

6. Procédé pour la préparation de composés de formule I selon la revendication 1, caractérisé par le fait qu'on fait réagir un amide de formule II 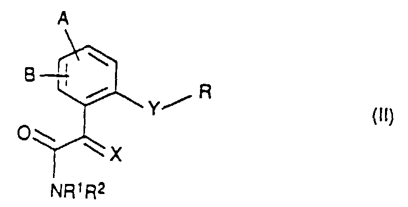 avec un agent de sulfuration.

7. Agent de lutte contre les parasites et agent de lutte contre les champignons, contenant un support inerte et un dérivé de thioamide de formule I selon la revendication 1 ou un dérivé de thioamide de formule I' selon la revendication 5.

8. Procédé pour lutter contre les champignons, caractérisé par le fait qu'on traite les champignons ou les matières, les plantes, les semences ou les sols menacés d'une attaque par les champignons avec une quantité efficace du point de vue fongicide d'un composé de formule I selon la revendication 1 ou d'un dérivé de thioamide de formule I' selon la revendication 5.

9. Procédé pour lutter contre les parasites, caractérisé par le fait qu'on fait agir une quantité efficace du point de vue insecticide, nématicide et/ou acaricide d'un thioamide de formule I selon la revendication 1 ou d'un dérivé de thioamide de formule I' selon la revendication 5 sur des insectes, nématodes ou mites ou sur leur biotope.

## Revendications (Revendications pour l'(les) Etat(s) contractant(s) suivant(s): AT, ES,GR, PT)

1. Thioamides de formule générale I, 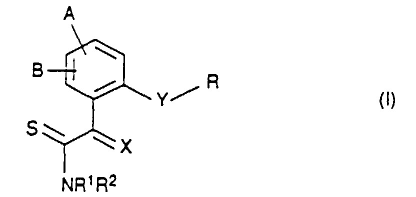 où les substituants ont la signification suivante:
A, B, R' hydrogène, cyano, alkyle en C₁-C₄, alcoxy en C₁-C₄ ou halogène,
X =CHCH₃ ou =N-OCH₃,
R¹, R² hydrogène ou alkyle en C₁-C₄,
Y -O-, -S-, -SO-, -SO₂-, -OCH₂-, -CH₂O-, -SCH₂-, -CH₂S-, -CH₂CH₂-, -CH=CH-, -C≡C- et -CH₂O-N=CR'-, et
R hydrogène, alkyle en C₁-C₁₀ éventuellement substitué, cycloalkyle en C₃-C₆ éventuellement substitué, aryle éventuellement substitué ou hétéroaryle éventuellement substitué, ou
tandis que l'expression "éventuellement substitué" désigne outre l'hydrogène égalememt un reste halogéno, cyano, nitro, CO₂-(alkyle en C₁-C₄), CO-(alkyle en C₁-C₄), alkyle en C₁-C₄, alcoxy en C₁-C₄, halogénoalkyle en C₁-C₄, alcoxyimino(C₁-C₄)-alkyle en C₁-C₄, aryle, aryloxy, benzyloxy, hétéroaryle, hétéroaryloxy, cycloalkyle en C₃-C₆, et
tandis que l'expression "hétéroaryle" désigne un hétérocycle aromatique, éventuellement substitué, à un, deux ou trois noyaux, penta- ou hexagonal.

2. Composés de formule I selon la revendication 1, dans lesquels X représente CHCH₃, R¹, A et B représentent l'hydrogène et R² représente un groupe méthyle.

3. Composés de formule I selon la revendication 1, dans lesquels X représente NOCH₃, R¹, A et B représentent l'hydrogène et R² représente un groupe méthyle.

4. Procédé pour la préparation de composés de formule I selon la revendication 1, caractérisé par le fait qu'on fait réagir un amide de formule II 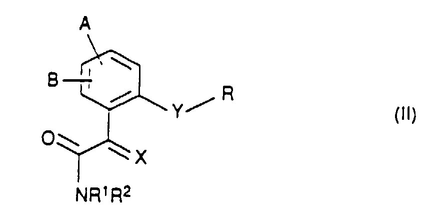 avec un agent de sulfuration.

5. Agent de lutte contre les parasites et agent de lutte contre les champignons, contenant un support inerte et un dérivé de thioamide de formule I selon la revendication 1.

6. Procédé pour lutter contre les champignons, caractérisé par le fait qu'on traite les champignons ou les matières, les plantes, les semences ou les sols menacés d'une attaque par les champignons avec une quantité efficace du point de vue fongicide d'un composé de formule I selon la revendication 1.

7. Procédé pour lutter contre les parasites, caractérisé par le fait qu'on fait agir une quantité efficace du point de vue insecticide, nématicide et/ou acaricide d'un thioamide de formule I selon la revendication 1 sur des insectes, nématodes ou mites ou sur leur biotope.

8. Composé de formule I selon la revendication 1, dans lequel Y représente CH₂O, R¹ représente un groupe méthyle, A, B et R² représentent l'hydrogène, R représente un groupe 2-méthylphényle et X représente NOCH₃.

9. Composé de formule I selon la revendication 1, dans lequel Y représente CH₂O, R¹ représente un groupe méthyle, A, B et R² représentent l'hydrogène, R représente un groupe 2,5-diméthylphényle et X représente NOCH₃.

10. Composé de formule I selon la revendication 1, dans lequel Y représente CH₂O, R¹ représente un groupe méthyle, A, B et R² représentent l'hydrogène, R représente un groupe 4-méthoxyiminométhylphényle et X représente NOCH₃.
